# EUROPEAN PATENT APPLICATION

(11) **EP 4 778 488 A1**
(43) Date of publication of application: **22.07.2026**
(21) Application number: 24855589.8
(22) Date of filing: 31.07.2024
(51) Int. Cl.: A61B 34/37, A61B 17/34, A61B 90/00, B25J 9/16

(54) **SURGICAL ROBOT SYSTEM**

(30) Priority: 23.08.2023 CN 202311067426
(71) Applicant: Shenzhen Edge Medical Co., Ltd., Shenzhen, Guangdong 518000 (CN)
(72) Inventor: YAN, Yusheng, Shenzhen, Guangdong 518000 (CN); LIN, Mincai, Shenzhen, Guangdong 518000 (CN); LUO, Ziming, Shenzhen, Guangdong 518000 (CN); YE, Guoqiang, Shenzhen, Guangdong 518000 (CN); WANG, Jianchen, Shenzhen, Guangdong 518000 (CN)
(74) Representative: Zacco Sweden AB
(86) International application number: PCT/CN2024/108818
(87) International publication number: WO 2025/039863

(57) **Abstract**

The present application discloses a surgical robot system. The surgical robot includes a controller, a surgical tool, and a trocar. The surgical tool is coupled to the controller and is extendably accommodated in an inner cavity of the trocar, and includes a flexible portion at a distal end. The control method includes: determining, in response to control information for the surgical tool, whether the flexible portion and the trocar are in a first or second relative positional relationship according to a length of the flexible portion accommodated in the trocar; performing motion control on the flexible portion according to the control information and in accordance with a preset first constraint associated with the first relative positional relationship; or, performing motion control on the flexible portion according to the control information and in accordance with a preset second constraint associated with the second relative positional relationship. The present application achieves different motion constraints on the flexible portion of the surgical tool at different positions relative to the trocar, preventing damage to the flexible portion during movement relative to the trocar.

## Description

This application claims priority to Chinese Patent Application No. CN 202311067426.4, filed with the China National Intellectual Property Administration on August 23, 2023, entitled "Surgical Robot System", the entire contents of which are incorporated herein by reference.

### TECHNICAL FIELD

The present application relates to the field of medical machinery technology, and in particular, to a surgical robot system.

### BACKGROUND

During the use of a surgical robot, surgical tools (including surgical instruments and endoscopes) are usually controlled by a chief surgeon sitting in front of a master console through a master-slave motion control mode to control a slave operation device to perform corresponding surgical actions. In order to improve the fluency of the surgical operation process, after the surgical tool is installed on a robotic arm of a patient surgical platform and activated by software, it can be handed over to the chief surgeon for master-slave control operation.

In existing solutions for slave operation devices, due to spatial constraints in the mechanical structural design of a trocar, the operable space of the surgical tool inside and outside the trocar is different, and therefore the corresponding applicable surgical scenarios are also different. Existing solutions do not configure constraint conditions for the motion of the surgical tool inside and outside the trocar, such that when an operator controls a flexible portion of the surgical tool to extend out of the trocar or retract into the trocar, the flexible portion may be in a bent state, causing a collision between the bent portion and a port of the trocar, resulting in damage to the flexible portion or the port of the trocar.

### SUMMARY

Embodiments of the present application provide a surgical robot system that can solve the technical problem of existing surgical tools having excessive degrees of freedom in both intra- and extra- trocar movements, which makes it easy to cause damage to the surgical tools during operation.

A first aspect of the present application provides a control method for a surgical robot, the surgical robot includes a controller, a surgical tool, and a trocar. The surgical tool is extendably accommodated and extendably accommodated within the trocar, and includes a flexible portion at a distal end. The surgical tool is extendably accommodated within the trocar, and includes a flexible portion at a distal end. The controller is configured to execute motion control of the surgical tool. The control method includes: determining, in response to control information for the surgical tool, whether the flexible portion and the trocar are in a first relative positional relationship or a second relative positional relationship; performing motion control on the flexible portion according to the control information and in accordance with a preset first constraint associated with the first relative positional relationship; or performing motion control on the flexible portion according to the control information and in accordance with a preset second constraint associated with the second relative positional relationship, where a length of the flexible portion accommodated within the trocar is shorter in the first relative positional relationship than in the second relative positional relationship.

A second aspect of the present application provides a surgical robot system, which includes the surgical robot as described above and a master console. The master console is communicatively connected to the surgical robot. The controller is configured to: determine, in response to control information sent from the master console, whether the flexible portion and the trocar are in a first relative positional relationship or a second relative positional relationship; perform motion control on the flexible portion according to the control information and in accordance with a preset first constraint associated with the first relative positional relationship; or perform motion control on the flexible portion according to the control information and in accordance with a preset second constraint associated with the second relative positional relationship, where a length of the flexible portion accommodated within the trocar is shorter in the first relative positional relationship than in the second relative positional relationship.

A third aspect of the present application provides a readable storage medium, wherein a computer program is stored on the readable storage medium, and the computer program, when executed by a processor, causes steps of the above control method for the surgical robot to be implemented.

Beneficial effects of the present application are as follows: When receiving the control information for the surgical tool, it is necessary to determine the relative positional relationship between the flexible portion at the distal end of the surgical tool and the trocar. In different relative positional relationships, the controller responds to the control information with different constraints and performs motion control on the flexible portion with different degrees of freedom of motion. For example, the flexible portion has more degrees of freedom of motion outside the trocar than inside the trocar, preventing damage caused by the flexible portion entering and exiting the trocar in a bending state. Meanwhile, the surgical tool performs master-slave operations with different constraints inside and outside the trocar, which can be suitable for surgical scenarios in narrow cavities.

### DESCRIPTION OF THE DRAWINGS

FIG. 1 is a schematic structural diagram illustrating a surgical robot system in the present application;
FIG. 2 is a schematic structural diagram illustrating a surgical robot in the present application;
FIG. 3 is a first flowchart illustrating a control method for a surgical robot in the present application;
FIG. 4 is a schematic structural diagram illustrating a surgical tool in the present application;
FIG. 5A is a schematic structural diagram illustrating a trocar coupled with a surgical tool in the present application;
FIG. 5B is another schematic structural diagram illustrating a trocar coupled with a surgical tool in the present application;
FIG. 6 is a first schematic diagram illustrating a position of a flexible portion relative to a trocar in the present application;
FIG. 7 is a second schematic diagram illustrating a position of a flexible portion relative to a trocar in the present application;
FIG. 8A is a first schematic diagram illustrating resetting of a remote center of motion on a trocar in the present application;
FIG. 8B is a second schematic diagram illustrating resetting of a remote center of motion on a trocar in the present application;
FIG. 8C is a third schematic diagram illustrating resetting of a remote center of motion on a trocar in the present application;
FIG. 9 is a second flowchart illustrating a control method for a surgical robot in the present application;
FIG. 10A is a third schematic diagram illustrating a position of a flexible portion relative to a trocar in the present application;
FIG. 10B is a fourth schematic diagram illustrating a position of a flexible portion relative to a trocar in the present application;
FIG. 11 is a fifth schematic diagram illustrating a position of a flexible portion relative to a trocar in the present application;
FIG. 12 is a schematic diagram illustrating a display interface in the present application;
FIG. 13 is another schematic diagram illustrating a display interface in the present application; and
FIG. 14 is a third flowchart illustrating a control method for a surgical robot in the present application.

### DETAILED DESCRIPTION OF THE EMBODIMENTS

To facilitate understanding of the present application, the present application will be described in more detail below with reference to the accompanying drawings and specific embodiments.

Preferred embodiments of the present application are given in the accompanying drawings. However, the present application may be implemented in many different forms and is not limited to the embodiments described in the present specification. On the contrary, the purpose of providing these embodiments is to make the understanding of the disclosure of the present application more thorough and comprehensive.

It should be noted that, unless otherwise defined, all technical and scientific terms used in the present specification have the same meanings as understood by persons skilled in the technical field to which the present application belongs. The terms used in the specification of the present application are merely for the purpose of describing specific embodiments, and are not intended to limit the present application. For example, the term "a plurality of" includes two or more.

### I. Surgical Robot System

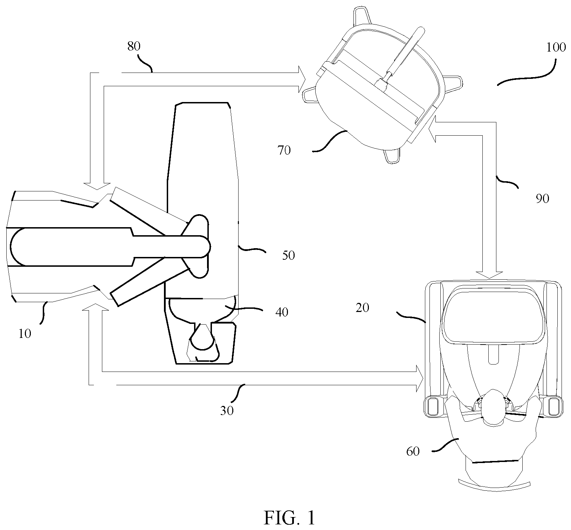

To facilitate understanding, the structure of the surgical robot will be described next. Referring to FIG. 1 to FIG. 2, the present application provides an embodiment of a surgical robot system, which is specifically shown as follows:
As shown in FIG. 1, a surgical robot system 100 at least includes a surgical robot 10 and a master console 20, and the master console 10 is communicatively connected to the surgical robot 20. In response to control information output by the master console, the surgical robot executes a master-slave operation according to the received control information, so as to complete a corresponding surgical action.

Specifically, in an exemplary surgical environment, the surgical robot 10 and the master console 20 are communicatively connected through a master-slave communication bus 30, and the control information issued by the master console 20 is transmitted to the surgical robot 10 through the master-slave communication bus 30. For an executed object of the surgical robot, such as a patient 40, placed on an operating table 50 according to a required posture, the surgical robot may be pushed and placed beside the operating table 50, and extends a robotic arm to a preset orientation of the patient 40 to perform a surgical operation. The master console 20 is manipulated by a doctor 60, and synchronously outputs the control information according to the operation of the doctor 60, where the master console 20 and the surgical robot 10 may be deployed in the same connected space or in completely isolated different spaces, so that the doctor can realize a remote surgical operation. The robotic arm is also coupled with an endoscope (not shown in the figure) for in-vivo intervention, which is used for observing tissue and a motion condition of a surgical tool during the surgical process. The tissue within the field of view of the endoscope is displayed through an imaging cart 70. The imaging cart 70 and the endoscope are communicatively connected through an endoscope data cable 80, and a video stream captured in real time is sent to the imaging cart 70 through the endoscope data cable for display. At the same time, when the doctor 60 controls the surgical robot 10 at the master console 20, the imaging cart forwards the displayed video stream to the master console 20 through a main image communication bus 90. The video stream is also presented in real time on the master console 20, providing the doctor 60 with a surgical field of view and the motion condition of the surgical tool.

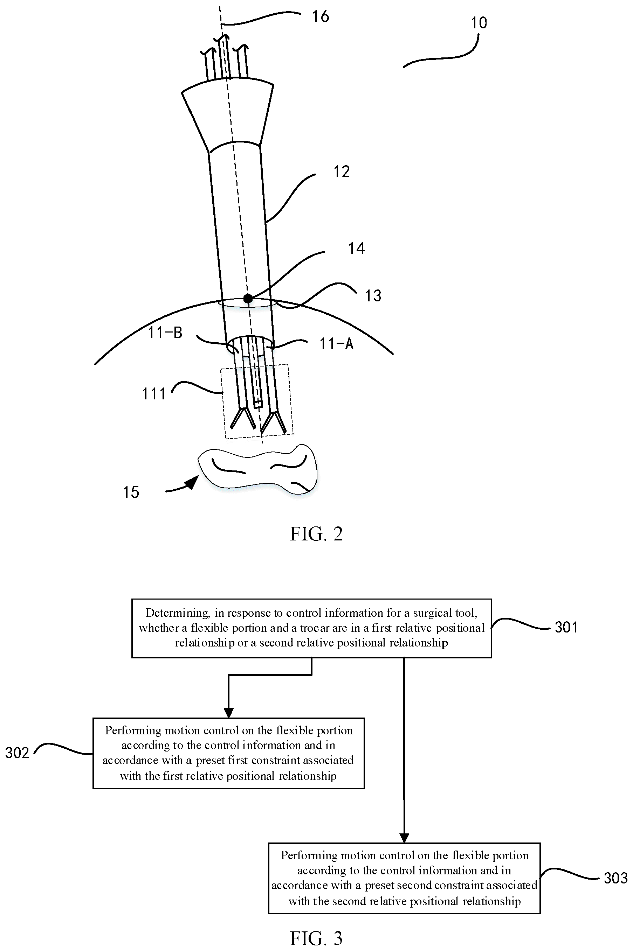

As shown in FIG. 2, the surgical robot 10 is used for actually executing the surgical action, and specifically includes a controller (not shown in the figure), a surgical tool 11 (a surgical tool 11-A, a surgical tool 11-B), and a trocar 12. The surgical tool 11 is coupled with the controller, and is extendably accommodated in an inner cavity of the trocar 12, and includes a flexible portion 111 at a distal end. The controller is configured to execute motion control of the surgical tool 11, and the control operations include: determining whether the flexible portion 111 and the trocar 12 are in a first relative positional relationship or a second relative positional relationship in response to the control information sent from the master console 10; performing motion control on the flexible portion 111 according to the control information and in accordance with a preset first constraint associated with the first relative positional relationship; or performing motion control on the flexible portion 111 according to the control information and in accordance with a preset second constraint associated with the second relative positional relationship.

In this embodiment, for each time the master console sends the control information to the surgical robot, the surgical robot will not directly execute the corresponding surgical action based on the control information. Instead, the surgical robot will first verify the relative positional relationship (the first relative positional relationship and the second relative positional relationship) of the flexible portion at a current moment, and different relative positional relationships are associated with corresponding motion constraints (the first constraint and the second constraint). Based on the determined motion constraint, the control information is adjusted, and a control instruction for actual execution is output, so as to actuate the flexible portion to execute the surgical action. That is, for the control information output by the master console, the surgical robot does not completely execute the surgical action in the control information.

Exemplarily, the first constraint includes a related constraint on an executable degree-of-freedom A and an executable degree-of-freedom B of the flexible portion, and the second constraint includes a related constraint on the executable degree-of-freedom B of the flexible portion. The control information output by the master console includes a first control instruction and a second control instruction, where the first control instruction indicates the flexible portion to execute the degree-of-freedom A, and the second control instruction indicates the flexible portion to execute the degree-of-freedom B. The surgical robot filters the control information such that, if the flexible portion is in the second relative positional relationship, the surgical robot does not respond to the first control instruction, but only to the second control instruction, allowing the flexible portion to execute the surgical action according to the degree-of-freedom B. If the flexible portion is in the first relative positional relationship, the surgical robot responds simultaneously to the first and second control instructions, allowing the flexible portion to execute the surgical action according to both the degree-of-freedom A and the degree-of-freedom B. Specifically, the control information, when transmitted to the surgical robot to control the surgical tool to execute the surgical action, at least includes orientation information and distance information for controlling the motion of the flexible portion. The orientation information, for example, represents advancing, retracting, and/or bending of the flexible portion along the inner cavity of the trocar, and the distance information, for example, represents an advancing amount, a retracting amount, and/or a bending angle of the flexible portion. By filtering the orientation information and the distance information, the resulting control instruction is ensured to meet the requirement of the first constraint or the second constraint.

In this embodiment, the first relative positional relationship and the second relative positional relationship may be determined based on the size of the movable space of the flexible portion relative to the trocar. The relative positional relationship is determined as the first relative positional relationship when the flexible portion has a relatively large movable space, and as the second relative positional relationship when the flexible portion has a relatively small movable space. The first constraint and the second constraint may also be preset according to structural characteristics of the flexible portion and the trocar, based on the size of the movable space after the two are coupled. For example, in the first constraint, a larger motion amplitude (such as more movable orientations and a larger movable distance) is set for the flexible portion, and in the second constraint, a smaller motion amplitude (such as fewer movable orientations and a smaller movable distance) is set for the flexible portion.

Exemplarily, the first relative positional relationship may be that the flexible portion is located outside the trocar, and the movable space of the flexible portion outside the trocar is larger. The second relative positional relationship may be that the flexible portion is located inside the trocar, and due to physical limitations of the trocar, the movable space of the flexible portion is smaller. Compared to the second constraint, the first constraint is not subject to the physical limitations of the trocar. Thus, the motion amplitude set by the first constraint is allowed to at least increase the radial extension range of the flexible portion outside the trocar.

In addition, in a specific surgical space, the trocar 12 is arranged at a human body incision 13 and disposed at a fixed position. Meanwhile, a remote center of motion 14 is further provided on the trocar 12, so that the trocar 12 and the surgical tool 11 (11-A, 11-B) can move around the remote center of motion 14. The surgical tool 11 (11-A, 11-B) coupled to the inner cavity of the trocar 12 can extend distally to reach a target tissue position (such as a lesion 15). The trocar 12 has a central axis 16, and the remote center of motion 14 is preferably provided at an intersection position of the central axis 16 and a center of the human body incision 13, so that when the trocar moves around the remote center of motion 14 along the central axis 16, damage to the tissue is prevented.

In addition, for the surgical tool 11 (11-A, 11-B) coupled to the inner cavity of the trocar shown in FIG. 2, to achieve hand-eye coordination during the surgical process, usually at least one endoscope 112-A and at least two surgical instruments 112-B are installed. When three or more surgical instruments are installed, the doctor needs to operate an instrument switching function on the master console to switch between different surgical tools.

### II. Master-Slave Operation - Sleeve-Hiding Function

The surgical robot of the present application has been described above. Next, referring to FIG. 2 to FIG. 7, a first embodiment of a control method for a surgical robot provided by the present application is shown, which specifically includes the following operations:
301: determining, in response to control information for the surgical tool, whether the flexible portion and the trocar are in a first relative positional relationship or a second relative positional relationship;
302: performing motion control on the flexible portion according to the control information and in accordance with a preset first constraint associated with the first relative positional relationship; or,
303: performing motion control on the flexible portion according to the control information and in accordance with a preset second constraint associated with the second relative positional relationship.

In this embodiment, the flowchart shown in FIG. 3 introduces the control method executed by the surgical robot in response to the control information sent from the master console in the surgical robot system. The previous embodiment regarding the surgical robot system has already introduced the control process at the single end of the surgical robot, which can be referred to the aforementioned content, and will not be repeated here. It should be noted that, in the control process of the entire surgical tool, it is at least necessary to execute the motion control according to the first constraint and the second constraint based on the first relative positional relationship and the second relative positional relationship of the flexible portion. As for other parts in the surgical tool, adaptive adjustments may also be made according to specific requirements, for example, different motion constraints may be configured according to the aforementioned first relative positional relationship and second relative positional relationship. Herein, the first relative positional relationship, compared to the second relative positional relationship, results in a shorter length of the flexible portion accommodated within the trocar.

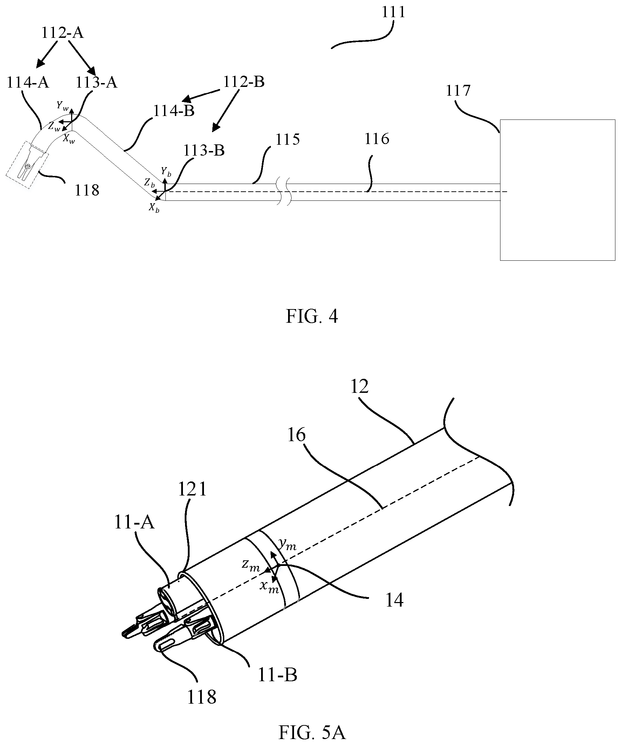

In one implementation, as shown in FIG. 4 which is a schematic diagram of a surgical tool, the flexible portion 111 at the distal end of the surgical tool includes at least one joint mechanism (112-A, 112-B), and the joint mechanism includes a base portion (113-A, 113-B) and a joint portion (114-A, 114-B), where the number of joint mechanisms (112-A, 112-B) constrained by the first constraint is greater than the number of joint mechanisms (112-A, 112-B) constrained by the second constraint, and the constraint on the joint mechanism (112-A, 112-B) includes a constraint on a motion degree of freedom of the joint portion (114-A, 114-B).

In this embodiment, the flexible portion realizes a flexible motion, such as a bending motion, through at least one joint mechanism. Further, the flexible motion is implemented by the joint portion, and a joint base serves as a reference, that is to say, related information such as orientation and distance included in the control information is based on the joint base to implement the motion control of the joint portion. And the motion degree of freedom of the joint portion increased in the first constraint relative to the second constraint is exactly the flexible motion of the joint portion.

Besides this, the surgical tool may also include a long-axis linkage 115 at a proximal end, which may be made of a rigid material. A central axis 116 of the surgical tool is parallel to or coincides with the central axis of the trocar. A drive unit 117 which is a placement location for active devices configured to control the long-axis linkage 115, the joint mechanism (112-A, 112-B), and the end effector 118 to execute motion. The long-axis linkage 115 is connected to the base portion (113-A, 113-B) and the drive unit 117, and can realize rotation motion around its own axis according to the control information, driving a rotation motion of the flexible portion, and perform axial translation along the central axis of the trocar, driving the flexible portion to perform axial translation.

In one implementation, for the motion of the end effector executing a functional operation, such as the opening and closing motion of an end clamping tool, the degree of freedom of the opening and closing motion is not affected by the first relative positional relationship or the second relative positional relationship, and the corresponding motion action can be executed in both cases. That is, both the first constraint and the second constraint encompass the motion degree of freedom of the end effector performing the functional operation. Moreover, the first constraint does not impose additional constraints on the motion degree of freedom of the end effector performing the functional operation compared to the second constraint.

Specifically, the motion control for the flexible portion at least includes two aspects, one is the motion control of the flexible portion based on the central axis, and the other is the motion control of the joint portion based on the base portion. The former, during the motion process of the flexible portion, will always keep the central axis of the surgical tool parallel to the central axis of the trocar, and the latter, during the motion process of the flexible portion, will cause the central axis of the joint portion to have at least a local axis deviating from the central axis of the trocar. That is, the first constraint at least includes the motion control of the flexible portion in these two aspects, and the second constraint at least includes the motion control of the flexible portion based on the central axis in the first aspect.

Exemplarily, the first type of motion control for the flexible portion includes the following three situations: the flexible portion performs axial translation along the central axis of the trocar along with the entire surgical tool; the flexible portion performs rotation along the flexible portion's own central axis along with the entire surgical tool; and the trocar performs rotation along the trocar's own central axis, driving the flexible portion to perform rotation along the central axis of the trocar along with the entire surgical tool. In all three aforementioned motion controls for the flexible portion, the central axis of the surgical tool remains parallel to the central axis of the trocar, and the pose configuration of the flexible portion here is a straightened configuration relative to the long-axis linkage.

Exemplarily, the second type of motion control for the joint portion includes: a bending motion of the joint portion along the base portion. As shown in FIG. 4, the joint portion 114-A of the joint mechanism 112-A bends downward towards the central axis of the trocar relative to the base portion 113-A, and the joint portion 114-B of the joint mechanism 112-B bends upward towards the central axis of the trocar relative to the base portion 113-B. The joint portion 114-A and the joint portion 114-B both form a preset angle with the central axis of the trocar.

Herein, the base portion 113-A is provided with a coordinate system XwYwZw, the base portion 113-B is provided with a coordinate system XbYbZb, and the joint portion 114-A and the joint portion 114-B perform bending motion along the coordinate system XwYwZw and the coordinate system XbYbZb, respectively. It should be noted that the provision of two coordinate systems, XwYwZw and XbYbZb, enables the independent control of the two joint mechanisms (112-A, 112-B) within the flexible portion.

In one implementation, as shown in FIG. 4 and FIG. 5A, the flexible portion also includes an end effector 118, and the joint mechanism includes a first joint mechanism 112-A and a second joint mechanism 112-B. The first joint mechanism 112-A includes a first base portion 113-A and a first joint portion 114-A. The second joint mechanism 112-B includes a second base portion 113-B and a second joint portion 114-B. The second joint portion 114-B is coupled with the first base portion 113-A, and the first joint portion 114-A is coupled with the end effector 118. The determining whether the flexible portion 111 and the trocar 12 are in the first relative positional relationship or the second relative positional relationship includes: determining whether at least one of the end effector 118, the first base portion 113-A, and the second base portion 113-B satisfies a preset positional relationship relative to a port position 121 of the trocar 12; determining that the flexible portion 111 and the trocar 12 are in the first relative positional relationship if at least one of the end effector 118, the first base portion 113-A, and the second base portion 113-B satisfies the preset positional relationship; and determining that the flexible portion 111 and the trocar 12 are in the second relative positional relationship if none of the end effector 118, the first base portion 113-A, and the second base portion 113-B satisfies the preset positional relationship.

In this embodiment, the purpose of evaluating the relative positional relationship between the flexible portion and the trocar is to select the motion constraint of the flexible portion under different movable spaces. Here, the distinction of the movable space of the flexible portion relative to the trocar is bounded by the position of the trocar's port. The difference between the preset first constraint and second constraint lies in increasing the flexible motion of the joint portion, and the flexible motion of the joint portion is an independent control of the joint portion based on the base portion. Therefore, the relative positional relationship between the trocar and the flexible portion can be evaluated through the relative position between the port position of the trocar and the base portion.

In addition, compared to the aforementioned content, the first constraint does not impose additional constraint on the motion degree of freedom of the end effector executing the functional operation with respect to the second constraint. In another implementation, it is also possible to evaluate the relative positional relationship between the end effector and the port position, and the first constraint may also increase the motion of the end effector relative to the second constraint, such as the opening and closing motion of the end effector having a clamping function. This prevents damage to the trocar or the surgical tool when the end effector executes a functional operation.

Specifically, the preset positional relationship may be that the end effector, the first base portion, or the second base portion is located outside the port position. Since the flexible portion sequentially includes, from the distal end to the proximal end, the end effector, the first base portion, and the second base portion, when the second base portion satisfies the preset positional relationship, the first base portion and the end effector also satisfy the preset positional relationship. This reasoning applies analogously.

In addition, as shown in FIG. 4, the first joint mechanism may be a wrist joint mechanism, and the second joint mechanism may be a shoulder-elbow joint mechanism. As described above, the coordinate system XwYwZw is established at a wrist base (the first base portion), and the coordinate system XbYbZb is established at a shoulder-elbow base (the second base portion). When a shoulder-elbow joint (the second joint portion) performs a flexible motion based on the shoulder-elbow base, the shoulder-elbow joint can realize a rotation motion with two degrees of freedom around an Xb axis and a Yb axis, thereby driving a translation motion with two degrees of freedom of the wrist base in the XwYw plane. When a wrist joint (the first joint portion) performs flexible motion based on the wrist base, the wrist joint can also realize rotation motion of two degrees of freedom around an Xw axis and a Yw axis, to perform posture adjustment on the end effector of the surgical instrument and a camera lens of the endoscope. During a motion process of the shoulder-elbow joint, a Zb axis of the coordinate system XbYbZb and a Zw axis of the coordinate system XwYwZw need to always remain parallel.

In this application scenario, in combination with the aforementioned control information including the orientation information and the distance information, such information may be further represented by two-degree-of-freedom information of the shoulder-elbow joint around the Xb axis and the Yb axis, and two-degree-of-freedom information of the wrist joint around the Xw axis and the Yw axis.

Herein, the long-axis linkage, the shoulder-elbow joint mechanism, and the wrist joint mechanism may be motion-decoupled, that is, the three separately execute motion control. The multiple joint mechanisms may also execute joint motion, for example, a joint motion between the shoulder-elbow joint mechanism and the wrist joint mechanism, which is based on the shoulder-elbow base, and can realize adjustment of poses (positions and postures) of each joint mechanism and the end effector under a shoulder-elbow base coordinate system.

Exemplarily, as shown in FIG. 5A, the surgical tool coupled to the inner cavity of the trocar 12 includes: an endoscope 11-A and two surgical instruments 11-B. The main structure configurations of the surgical instrument 11-B and the endoscope 11-A are the same, and the difference lies in that the end effectors 118 are respectively a functional tool for clamping/ablation, and a camera.

Herein, a coordinate system XmYmZm is established at the remote center of motion 14 on the trocar central axis 16, the wrist joint mechanisms and the shoulder-elbow joint mechanisms of the three surgical tools are all inside the trocar and do not exceed the port position of the trocar, while the end effectors of the two surgical instruments are located outside the port position of the trocar. At this time, the endoscope 11-A and the surgical instrument 11-B as a whole may follow the trocar 12 to execute overall rotation motion around the remote center of motion 14, and may execute rotation motion around their own central axes and forward-backward translation motion along the trocar's central axis 116, and at the same time, the end clamping tool may execute opening and closing motion.

Herein, during the motion process of the surgical tool, the Zm axis of the coordinate system XmYmZm may also always remain parallel to the Zb axis of the coordinate system XbYbZb and the Zw axis of the coordinate system XwYwZw.

In this application scenario, in combination with the aforementioned control information including orientation information and distance information, such information may be further represented by rotational degree-of-freedom information and translational degree-of-freedom information of the surgical tool around the surgical tool's own axis.

In addition, the installation mode of the surgical tool as shown in FIG. 5A may be an initial installation state in which the surgical tool is accommodated in the trocar, each joint mechanism is at a software zero position, and subsequent motion of each joint mechanism are based on the zero position to perform bending motion. Through this initial installation state, the surgical tool applies the motion manner of responding to the control information as described above, allowing the endoscope 11-A and the surgical instrument 11-B to gradually extend out to gradually unlock the second constraint related to each joint mechanism. This is suitable for a surgical scenario where operations are carried out in a narrow surgical space, or a surgical scenario that requires the use of surgical instruments to create a surgical pathway in a case of no surgical space.

In one implementation, the initial installation state of the surgical tool in the trocar, in addition to the installation mode of the surgical tool as shown in FIG. 5A, may also be other installation modes, such as the end effectors of the endoscope 11-A and the surgical instrument 11-B are also retracted into the trocar. However, the prerequisite condition that needs to be observed is: the first joint mechanism, the second joint mechanism, and the long-axis linkage should not be disposed outside the trocar, or partially outside.

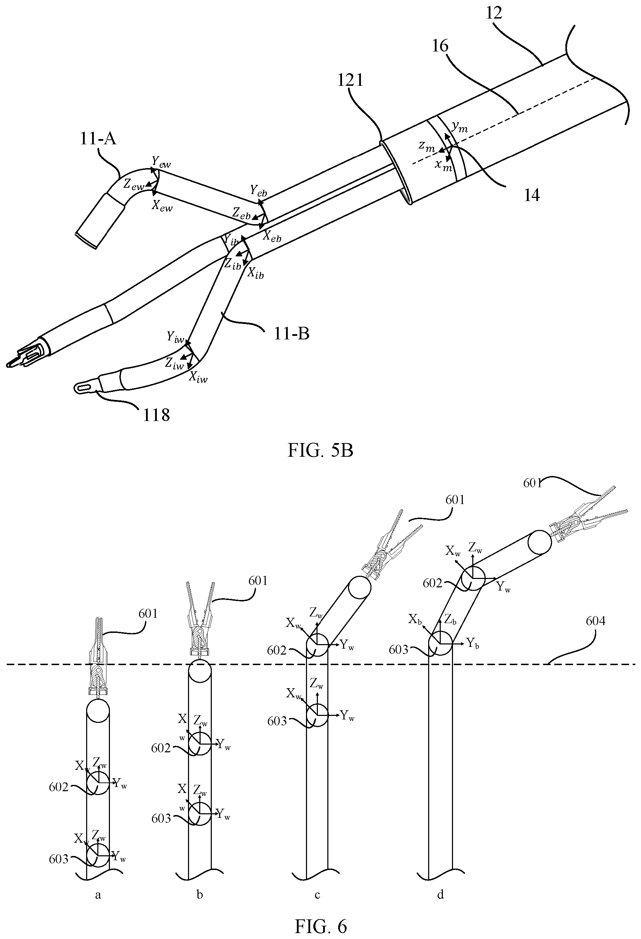

As shown in FIG. 5B, the shoulder-elbow bases and the wrist bases of the surgical instrument 11-B and the endoscope 11-A are all located outside the port position 121 of the trocar, this relative positional relationship belongs to the first relative positional relationship. The motion of each joint mechanism is not limited by the trocar (that is, belonging to the first constraint), the surgical instrument 11-B can achieve a surgical operation with six degrees of freedom in the operating space, and the endoscope 11-A can perform visual field exploration with six degrees of freedom in the operating space. The six degrees of freedom include: two degrees of freedom of rotation motion and translation motion of the surgical tool around the surgical tool's own axis, two degrees of freedom of the shoulder-elbow joint rotating around the Xb axis and the Yb axis, and two degrees of freedom of the wrist joint rotating around the Xw axis and the Yw axis. The first constraint includes the motion constraint of the six degrees of freedom.

Compared to the second constraint, the first constraint increases at least the constraint of the two degrees of freedom of the shoulder-elbow joint rotating around the Xb axis and the Yb axis, and the two degrees of freedom of the wrist joint rotating around the Xw axis and the Yw axis. That is, the second constraint includes: the motion constraint of the two degrees of freedom of rotation motion and translation motion of the surgical tool around the surgical tool's own axis.

In one implementation, the control information includes at least one degree of freedom information among the aforementioned six degrees of freedom, representing the orientation information and the distance information for controlling the motion of the flexible portion. The control information is filtered, so that the motion information finally output to the controller to actuate the flexible portion satisfies the first constraint or the second constraint.

Exemplarily, when filtering the control information, for instance, if the control information includes two degrees of freedom of the shoulder-elbow joint rotating around the Xb axis and the Yb axis, as well as the rotation motion around its own axis, then the information of the two degrees of freedom of the shoulder-elbow joint rotating around the Xb axis and the Yb axis will be screened out when the flexible portion is in the second relative positional relationship. That is, the control instructions for these two degrees of freedom will not be responded to, and only the control instruction for the rotation motion around its own axis will be responded to, so as to actuate the long-axis linkage to rotate around its own axis and drive the rotation of the flexible portion.

Furthermore, by controlling the endoscope to perform forward-backward translation motion along the central axis of the trocar, the field of view of the endoscope can be continuously adjusted in size, so that the wrist joint and the shoulder-elbow joint of the surgical instrument are covered within the field of view, thus assisting the surgical instruments to avoid colliding with each other during the operation process. Similarly, by controlling the surgical instrument to perform forward-backward translation motion along the central axis of the trocar, the operation distance between the surgical instrument and the lesion and human tissue can be adjusted.

In one implementation, the first constraint includes a first degree-of-freedom, a second degree-of-freedom constraint, and a third degree-of-freedom constraint. The operation of performing motion control on the flexible portion according to the control information and in accordance with the preset first constraint associated with the first relative positional relationship includes: performing motion control on the flexible portion according to the control information and in accordance with the first degree-of-freedom if the end effector satisfies the preset positional relationship, and the first base portion does not satisfy the preset positional relationship; performing motion control on the flexible portion according to the control information and in accordance with the second degree-of-freedom constraint if the first base portion satisfies the preset positional relationship, and the second base portion does not satisfy the preset positional relationship; and performing motion control on the flexible portion according to the control information and in accordance with the third degree-of-freedom constraint if the second base portion satisfies the preset positional relationship.

In this embodiment, as shown in FIG. 6, a simplified schematic diagram of four relative positional relationships between the flexible portion and the trocar is illustrated (as shown in a to d in FIG. 6). Herein, the preset positional relationship is that the end effector 601, the first base portion 602, and the second base portion 603 are located outside the port position 604 (equivalent to an area above the port position 604 shown in FIG. 6).

Specifically, for a in FIG. 6, the end effector 601, the first base portion 602, and the second base portion 603 are all located at an inner side of the port position 604, that is, none of the three satisfies the preset positional relationship. In this case, the flexible portion and the trocar are in the second relative positional relationship. For b in FIG. 6, the end effector 601 is located outside the port position 604, and the first base portion 602 and the second base portion 603 are both located at the inner side of the port position 604, then the first degree-of-freedom is used to perform motion control on the flexible portion. For c in FIG. 6, the end effector 601 and the first base portion 602 are both located outside the port position 604, and the second base portion 603 is located at the inner side of the port position 604, then in this case, the second degree-of-freedom constraint is used to perform motion control on the flexible portion. For d in FIG. 6, the end effector 601, the first base portion 602, and the second base portion 603 are all located outside the port position 604, then the third degree-of-freedom constraint is used to perform motion control on the flexible portion.

It should be noted that, as shown in FIG. 6, when the first joint portion and/or the second joint portion performs bending motion, the coordinate axes Zw and Zb of the first base portion and the second base portion still remain in a parallel state, and preferably the coordinate axes Zw and Zb remain parallel to the central axis of the surgical tool, or to the central axis of the trocar.

In one implementation, the first degree-of-freedom includes: the motion of the end effector executing the functional operation, and the rotation motion of the first joint portion and the second joint portion around their own central axes, and the rotation motion and translation motion along the central axis of the trocar. The second degree-of-freedom constraint includes: the first degree-of-freedom, and a bending motion of the first joint portion along the first base portion. The third degree-of-freedom constraint includes: the first degree-of-freedom, the second degree-of-freedom constraint, and a bending motion of the second joint portion along the second base portion.

Specifically, for b in FIG. 6, motion control is performed on the flexible portion according to the first degree-of-freedom, and the first degree-of-freedom includes the motion degree of freedom of the end effector executing the functional operation, and the rotational degree of freedom and translational degree of freedom of the first joint portion and the second joint portion around their own axes, and their own axes may be the Zw axis of the first base portion and the Zb axis of the second base portion. For c in FIG. 6, the second degree-of-freedom constraint further includes: two rotational degrees of freedom of the first joint portion around the Xw axis and the Yw axis in the second base portion. For d in FIG. 6, the third degree-of-freedom constraint further includes: two rotational degrees of freedom of the second joint portion around the Xb axis and the Yb axis in the second base portion.

In one implementation, for a in FIG. 6, the second constraint includes: the rotation motion of the end effector, the first joint portion, and the second joint portion around their own central axes, and the rotation motion and translation motion along the central axis of the trocar.

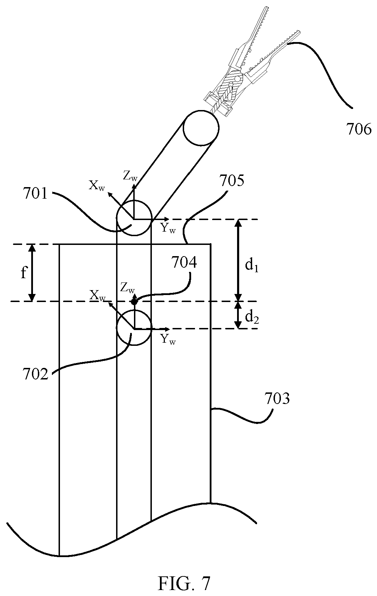

In one implementation, as shown in FIG. 7, the first base portion 701 is provided with a first coordinate system XwYwZw, the second base portion 702 is provided with a second coordinate system XbYbZb, a remote center of motion 704 is disposed on the central axis of the trocar 703, and the remote center of motion 704 has a preset distance threshold f from the port position 705. The determining whether at least one of the end effector 706, the first base portion 701, and the second base portion 702 satisfies the preset positional relationship relative to the port position of the trocar includes: determining first axial distances (d1, d2) along a distal direction of the central axis of the trocar between the first coordinate system XwYwZw and the second coordinate system XbYbZb and the remote center of motion 704, respectively; comparing a magnitude between each of the first axial distances (d1, d2) and the preset distance threshold f, and based on a result of the comparison, respectively determining whether the end effector 706, the first base portion 701, and the second base portion 702 satisfy the preset positional relationship relative to the port position 705 of the trocar 703.

In this embodiment, for example, if the first axial distance d1 between the first coordinate system XwYwZw and the remote center of motion 704 is greater than the preset distance threshold f, then it is determined that the first base portion 701 satisfies the preset positional relationship. If the first axial distance d2 between the second coordinate system XbYbZb and the remote center of motion 704 is smaller than the preset distance threshold f, then it is determined that the second base portion 702 does not satisfy the preset positional relationship.

Further, the straightened length d3 of the first joint portion is also known. When the first axial distance d1 is smaller than the preset distance threshold f, it is further determined whether d1 + d3 is greater than the preset distance threshold f. If d1 + d3 is greater than the preset distance threshold f, it is determined that the end effector 706 satisfies the preset positional relationship. If d1 + d3 is smaller than or equal to the preset distance threshold f, it is determined that the end effector 706 does not satisfy the preset positional relationship.

Exemplarily, the relative positions of the first coordinate system and the second coordinate system with respect to the remote center of motion may be represented by the first axial distance with positive and negative numbers. If the first coordinate system and the second coordinate system are located in an orientation of the remote center of motion towards the distal end, the first axial distances of the two with respect to the remote center of motion are represented by positive numbers. If the first coordinate system and the second coordinate system are located in an orientation of the remote center of motion toward the proximal end, the first axial distances of the two with respect to the remote center of motion are represented by negative numbers. For example, the first axial distance d1 between the first coordinate system XwYwZw and the remote center of motion 704 is a positive number, and the first axial distance d2 between the second coordinate system XbYbZb and the remote center of motion 704 is a negative number.

In a preferred technical solution, a spatial coordinate system may be constructed relative to the surgical robot, and position coordinates of the remote center of motion, the first coordinate system, and the second coordinate system in the spatial coordinate system are determined. Distances from the remote center of motion to the first coordinate system and the second coordinate system are respectively calculated based on the position coordinates. The first relative positional relationship or the second relative positional relationship between the flexible portion and the trocar is determined based on the magnitude between the distances and the preset distance threshold. Herein, the first relative positional relationship indicates that the flexible portion is in a non-sleeve-hiding state of the trocar, and the second relative positional relationship indicates that the flexible portion is in a sleeve-hiding state of the trocar. In the non-sleeve-hiding state, the first constraint having more motion degree-of-freedom constraints is applied to control the motion of the flexible portion, and in the sleeve-hiding state, the second constraint having fewer motion degree-of-freedom constraints is applied to control the motion of the flexible portion. In the sleeve-hiding state and the non-sleeve-hiding state, the control information is filtered to realize the control of the flexible portion with different degree-of-freedom constraints, which is exactly the sleeve-hiding function realized for the flexible portion relative to the trocar under the master-slave control of the master console over the surgical robot.

Herein, the first constraint increases the degree of freedom of a bending motion for at least one joint mechanism in the flexible portion relative to the second constraint, so that the transition of the flexible portion between the sleeve-hiding state and the non-sleeve-hiding state can avoid the bending of the joint mechanism from damaging the trocar and the flexible portion. Herein, the bending motion of the plurality of joint mechanisms can be independently controlled based on their respective degree-of-freedom constraints, enabling more flexible manipulation of the flexible portion for lesion treatment. This enhances applicability across a broader range of scenarios, such as operations in a narrow surgical space, creating a surgical pathway by operating surgical instruments when no pre-existing surgical space is available, or treating a lesion portion relatively close to the body surface.

### III. Sleeve-Hiding Function - Resetting Remote Center of Motion

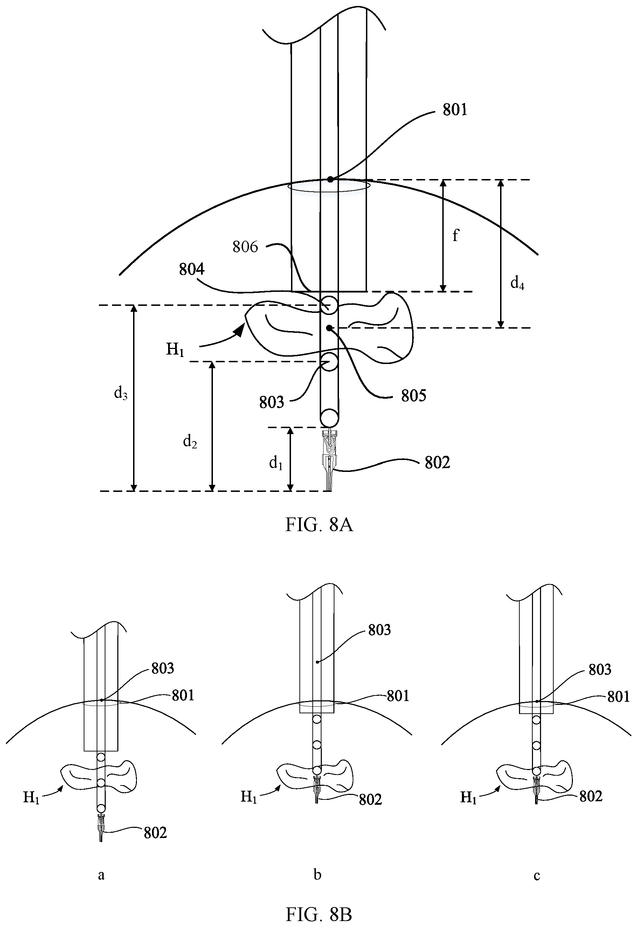

Referring to FIGS. 8A to 8C, a second embodiment of a control method for a surgical robot is provided in the present application, which is specifically illustrated as follows:
As shown in FIG. 8A, the control method involves acquiring size information (d1, d2, d3) of the flexible portion, and determining a second axial distance d4 between a target lesion region H1 and a remote center of motion 801; controlling the trocar to perform a translational translation motion relative to the central axis based on each of the size information (d1, d2, d3), the preset distance threshold f and the second axial distance d4; and resetting, in response to a setting instruction for the remote center of motion 801, the remote center of motion 801 according to a termination position of the trocar when performing the translational translation motion.

In this embodiment, the entire trocar accommodating the surgical tool is subjected to the translational translation motion control, so that when the end effector, the first base portion, or the second base portion translates to just be in the first relative positional relationship with the trocar, the motion limit of the proximal ends of the end effector, the first joint portion, or the second joint portion can reach the target lesion region. At this time, the doctor inputs the setting instruction for the remote center of motion at the master console to reset the remote center of motion on the trocar.

Still referring to FIG. 8A, respective segment lengths (d1, d2, d3) of the end effector 802, the first base portion 803 and the second base portion 804 may be determined according to the size information. According to each of the segment lengths (d1, d2, d3) and the second axial distance d4, a translation range of the trocar relative to the central axis of the trocar is determined, and the trocar is controlled to move within the translation range. Alternatively, according to each of the segment lengths (d1, d2, d3) and the second axial distance d4, respective translation nodes of the trocar relative to the central axis of the trocar are established, and the trocar is controlled to move at each of the translation nodes, so that the termination position of the trocar is coincides with one of the translation nodes.

Specifically, by taking a proximal endpoint 805 on the target lesion site, and determining the segment length d1 of the end effector 802, the segment length d2 of the first base portion 803 and the segment length d3 of the second base portion 804 based on the size information of the flexible portion, it is determined that when the end effector is just in the first relative positional relationship with the trocar, its distance to the target lesion region is required to be greater than or equal to the segment length d1, so that the motion limit of the proximal end of the end effector can reach the target lesion region. Similarly, it can be obtained that the distance from the first base portion to the target lesion region is required to be greater than or equal to the segment length d2, and the distance from the second base portion to the target lesion region is required to be greater than or equal to the segment length d3.

Following the above, since the end effector 802, the first base portion 803 and the second base portion 804 are just in the first relative positional relationship with the trocar, the segment length d1, the segment length d2 and the segment length d3 may also be determined as: the distances of the port position 806 to the proximal endpoint 805 respectively when the motion limit of the proximal ends of the end effector 802, the first base portion 803 and the second base portion 804 can reach the target lesion region.

Further, when the end effector 802, the first base portion 803 and the second base portion 804 are just in the first relative positional relationship with the trocar, the operation of determining the translation range of the trocar relative to the central axis of the trocar specifically includes:
determining, when the end effector 802 is just in the first relative positional relationship with the trocar, that a first translation range R1 is: R1 ≥ f+d1-d4;
determining, when the first base portion 803 is just in the first relative positional relationship with the trocar, that a second translation range R2 is: R2 ≥ f+d2-d4;
determining, when the second base portion 804 is just in the first relative positional relationship with the trocar, that a third translation range R3 is: R3 ≥ f+d3-d4; and
further determining a fourth translation range R4 to be: R4 < f, to ensure that the trocar is still in the patient's body after translation.

In one implementation, based on the aforementioned R1, R2, R3, and R4, a final translation range R of the trocar relative to the central axis of the trocar is defined as: (f+d1-d4 f+d1-d4 | f+d1-d4) ≤ R ≤ f. By operating on the master console, the trocar can perform arbitrary translation motion within the translation range R.

In another implementation, respective translation nodes of the trocar relative to the central axis of the trocar are determined (the translation lengths are respectively: f+d1-d4, f+d1- d4, f+d1-d4). Through the operation on the master console, the trocar is allowed to perform a jumping translational translation motion on each translation node, and at this point, the port position coincides with the translation node. Further, forward and backward translation buttons are configured in advance, in response to a touch operation on the forward translation button, the port position of the trocar is controlled to move to an adjacent translation node closer to the distal end, and in response to a touch operation on the backward translation button, the port position of the trocar is controlled to move to an adjacent translation node more closer to the proximal end.

By setting the translation range or the translation nodes of the trocar during the translational translation motion, on the one hand, the trocar is prevented from exiting the body, and on the other hand, when the surgical tool passes through the inner cavity of the trocar and enters the patient's body, at least one of the end effector, the first base portion and the second base portion extends out of the trocar, and at least one of the end effector, the first joint portion and the second joint portion can perform motion in accordance with the first constraint, to allow the end effector to process the target lesion region. It should be noted that the configuration of the translation range or the translation nodes also benefits from the fact that when the end effector, the first joint mechanism and the second mechanism are outside the trocar, these parts can be independently controlled according to the first constraint, and the surgical operation is realized outside the trocar in a partial sleeve-hiding manner.

In one implementation, the operation of resetting, in response to the setting instruction for the remote center of motion, the remote center of motion according to the termination position of the trocar when performing the translational translation motion includes: determining, in response to the setting instruction for the remote center of motion, a translation distance of the trocar according to the termination position of the trocar when performing the translational translation motion; controlling the remote center of motion to move according to the translation distance to obtain a reset remote center of motion, wherein the remote center of motion is located within a position range where the trocar is located.

In this embodiment, as shown in FIG. 8B, when the target lesion region is located at a position close to the human body incision 801, since the motion limit of the proximal end of the end effector 802 is located at a deeper position than the target lesion region H1, the target lesion region H1 cannot be processed (see a in FIG. 8B), so it is required to move the trocar to make the depth of the motion limit of the proximal end of the end effector 802 shallower until the end effector 802 reaches the target lesion region H1 (see b in FIG. 8B). By controlling the trocar to move a distance D in translation, the remote center of motion 803 is translated in the reverse direction by the distance D, resulting in the reset position of the remote center of motion 803 (see c in FIG. 8B), and meanwhile, under the constraint of the translation range or the translation nodes, the remote center of motion 803 remains within the position range of the trocar, which is equivalent to the trocar being located within the body surface of the patient.

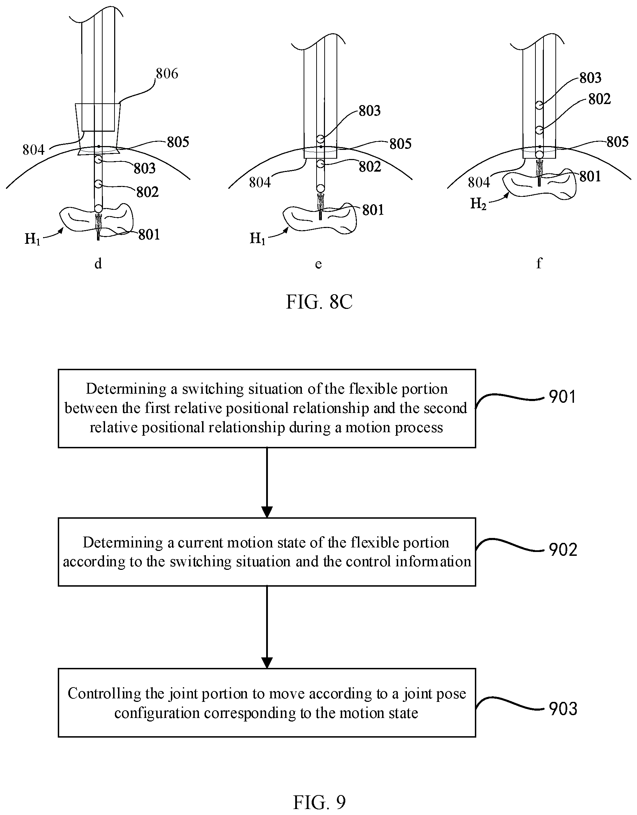

In this embodiment, as shown in FIG. 8C, in the flexible portion of the surgical tool, if the end effector 801, the first joint mechanism 802, and the second joint mechanism 803 cannot be independently controlled, it is required to extend all three of them out of the port position 804 of the trocar in order for the end effector 801 to process the target lesion region H1, and when the trocar translates to a limit position about to exit the human body incision 805, the motion limit of the proximal end of the end effector 801 is still at a deeper place relative to the target lesion region H1, and the target lesion region H1 cannot be processed. In this case, it is required to further translate the trocar to exit the body, and at the same time, a protective sleeve 806 is further provided between the trocar and the human body incision 805 to prevent the environment from causing infection to the patient through the human body incision 805 (see d in FIG. 8C).

In this embodiment, since the independent control is realized for the end effector 801, the first joint mechanism 802, and the second joint mechanism 803, it is unnecessary to extend all three of them out of the trocar, and extending only one of them is sufficient to enable the end effector 801 to treat the target lesion region H1. Based on this, the trocar can retract a shorter distance within the translation range, allowing the motion limit of the proximal end of the end effector 801 to reach the target lesion region H1/H2 without requiring the trocar to exit the body. This enables the end effector 801 to treat the target lesion region H1/H2 while reducing the risk of causing infection to the patient (see e and f in FIG. 8C).

Further, as the target lesion region is positioned closer to the body surface, the retraction distance of the trocar is adjusted. For example, the target lesion region target lesion region H1 shown in f of FIG. 8C is closer to the body surface position relative to the target lesion region target lesion region H2 shown in d of FIG. 8C, enabling control over the extension of one or more of the end effector 801, the first joint mechanism 802, and the second joint mechanism 803, This approach maximizes the motion degree of freedom of the flexible portion while ensuring that the trocar does not need to exit the body to reduce the risk of causing infection to the patient. For instance, the flexible portion depicted in e of FIG. 8C can realize two rotational degrees of freedom of the first joint portion and the translational and rotational degrees of freedom along the central axis (four degrees of freedom), allowing the end portion to perform functional operations with degrees of freedom in motion. However, the flexible portion depicted in f of FIG. 8C can only realize the translational and rotational degrees of freedom along the central axis (two degrees of freedom).

In addition, it is further detected whether a difference between the translation distance of the trocar and a preset distance threshold is within a preset difference threshold. If so, the trocar can be controlled to advance toward the distal direction so that the difference exceeds the preset difference threshold, preventing the port position of the trocar from just fitting on the human body incision, which would cause damage to the human body incision or the tissue portion in contact with the trocar when the relative radial displacement between the trocar and the patient is too large.

### IV. Sleeve-Hiding Function - Retraction and Restoration

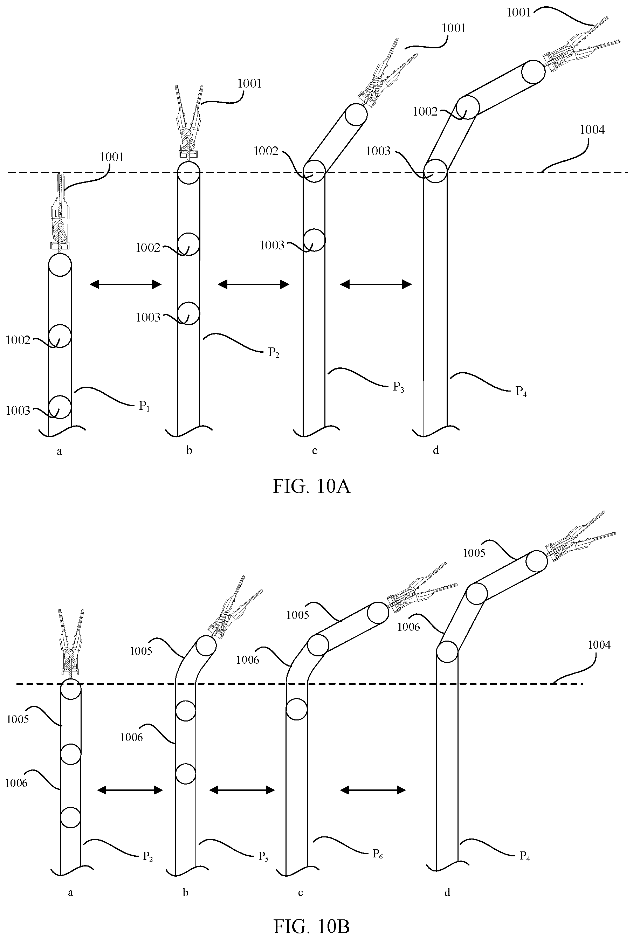

Referring to FIG. 9, FIG. 10A, and FIG. 10B, a third embodiment of a control method for a surgical robot is provided in the present application, which specifically includes the following operations:
901: determining a switching situation of the flexible portion between the first relative positional relationship and the second relative positional relationship during a motion process;
902: determining a current motion state of the flexible portion according to the switching situation and the control information; and
903: controlling the joint portion to move according to a joint pose configuration corresponding to the motion state.

In this embodiment, the first relative positional relationship of the flexible portion includes at least one of the end effector, the first base portion, and the second base portion satisfying the preset positional relationship, which is specifically represented as at least one being located outside the port position of the trocar. When the end effector is configured to satisfy the preset positional relationship, a relationship between the flexible portion and the trocar belongs to a first relationship. When the first base portion is configured to satisfy the preset positional relationship, a relationship between the flexible portion and the trocar belongs to a second relationship. When the second base portion is configured to satisfy the preset positional relationship, a relationship between the flexible portion and the trocar belongs to a third relationship. The first relative positional relationship includes the first relationship, the second relationship, and the third relationship. The operation of determining the switching situation of the flexible portion between the first relative positional relationship and the second relative positional relationship includes: determining a sequential switching situation among the first relationship, the second relationship, the third relationship, and the second relative positional relationship.

Specifically, as shown in a to d of FIG. 10A, which sequentially represent the positions of the flexible portion relative to the trocar under the second relative positional relationship, the first relationship, the second relationship, and the third relationship. Therefore, the first relative positional relationship is obtained when the flexible portion, in the second relative positional relationship, extends distally through the inner cavity of the trocar.

Further, when the relative positional relationship between the flexible portion and the trocar is switched from the second relative positional relationship to the first relative positional relationship for the first time, it is determined that the current motion state of the flexible portion belongs to a first type of state. When switching from the first relative positional relationship to the second relative positional relationship, or switching back from the second relative positional relationship to the first relative positional relationship again, it is determined that the current motion state of the flexible portion belongs to a second type of state. The motion state includes the first type of state and the second type of state. Then, combined with the translation motion information relative to the central axis in the control information, a final motion state of the first type of state or the second type of state is determined.

In one implementation, as shown in FIG. 10A, when controlling the motion of the joint portion based on a final configuration, the specific motion control manners are as follows:
1) Determining, based on the first type of state and the control information being an advancing control, a current first motion state of the flexible portion; and controlling the joint portion by completely mapping the control information to a real-time joint pose configuration based on the first motion state.
2) Determining, based on the first type of state and the control information being a retracting control, a current second motion state of the flexible portion; and controlling the joint portion in a straightened configuration P1 based on the second motion state.
3) Determining, based on the second type of state and the control information being an advancing control, a current third motion state of the flexible portion; and controlling the joint portion according to a memory configuration (P2 to P4) based on the third motion state.
4) Determining, based on the second type of state and the control information being a retracting control, a current fourth motion state of the flexible portion; and controlling the joint portion in the straightened configuration P1 based on the fourth motion state.

Herein, if the motion control manner 2) or the motion control manner 4) transitions from the retracting control to the advancing control, then they respectively enter the motion control manner 1) and the motion control manner 4).

In one implementation, the operation of determining the current motion state of the flexible portion according to the switching situation and the control information includes: determining whether the switching situation satisfies a preset condition, where the preset condition includes that the flexible portion currently performs a gradual switching between the first relative positional relationship and the second relative positional relationship, and prior to this, there exists a switching of the flexible portion from the second relative positional relationship to the first relative positional relationship; and determining the current motion state of the flexible portion according to the control information if the switching situation satisfies the preset condition.

In this embodiment, when the switching situation satisfies the preset condition, the current motion state of the flexible portion belongs to the aforementioned second type of state, that is, the flexible portion relative to the trocar is: switching from the second relative positional relationship to the first relative positional relationship again (a restoration state), or switching from the first relative positional relationship to the second relative positional relationship (a retraction state). Thus, it can be determined that prior to this, the flexible portion is required to first switch from the second relative positional relationship to the first relative positional relationship. In this case, according to the control information being the advancing control or the retracting control, the aforementioned motion control manner 3) or motion control manner 4) is utilized to perform motion control on the joint portion.

Specifically, regarding "prior to this, there exists the switching of the flexible portion from the second relative positional relationship to the first relative positional relationship", which means the position of the flexible portion relative to the trocar has previously switched from the second relative positional relationship (see a in FIG. 10A) to the second relationship (see b in FIG. 10A), or even further switched to the third relationship (see c in FIG. 10A) or the fourth relationship (see d in FIG. 10A). That is, prior to this, at least one of the end effector, the first base portion, and the second base portion extends outside the port position of the trocar.

Referring to FIG. 10A, when the flexible portion subsequently gradually switches between the first relative positional relationship and the second relative positional relationship, the switching manners include:
1) Gradually moving from the end effector 1001 starting to be exposed from the port position 1004 to the end effector 1001 being completely exposed from the port position 1004; (i.e., switching from a in FIG. 10A to b in FIG. 10A.)
2) Gradually moving from the end effector 1001 being completely exposed from the port position 1004 to the first base portion 1002 being completely exposed from the port position 1004; (i.e., switching from c in FIG. 10A to c in FIG. 10A.)
3) Gradually moving from the first base portion 1002 being completely exposed from the port position 1004 to the second base portion 1003 being completely exposed from the port position 1004; (i.e., switching from c in FIG. 10A to d in FIG. 10A.)
4) Gradually moving from the second base portion 1003 starting to retract back into the port position 1004 to the second base portion 1003 completely retracting back into the port position 1004; (i.e., switching from d in FIG. 10A to c in FIG. 10A.)
5) Gradually moving from the first base portion 1002 starting to retract back into the port position 1004 to the first base portion 1002 completely retracting back into the port position 1004; (i.e., switching from c in FIG. 10A to b in FIG. 10A.)
6) Gradually moving from the end effector 1001 starting to retract back into the port position 1004 to the end effector 1001 completely retracting back into the port position 1004. (i.e., switching from b in FIG. 10A to a in FIG. 10A.)

In one implementation, the operation of controlling the joint portion to move according to the joint pose configuration corresponding to the motion state includes: setting, when the control information is the advancing control, the joint pose configuration corresponding to the motion state as a memory configuration of the joint portion when the flexible portion in the first relative positional relationship is about to switch to the second relative positional relationship; setting, when the control information is the retracting control, the joint pose configuration corresponding to the motion state as a straightened configuration of the joint portion; and controlling the joint portion to move according to the memory configuration or the straightened configuration.

In this embodiment, when the control information is the advancing control, the flexible portion is subjected to the aforementioned switching manner 1) to switching manner 3). When the control information is the retracting control, the flexible portion is subjected to the aforementioned switching manner 4) to switching manner 6). The memory configuration of the joint portion is a pose configuration of the joint portion when the flexible portion previously switched from the second relative positional relationship to the first relative positional relationship. Herein, the previous pose configuration of the joint portion further includes: the pose configuration of the joint portion at the most recent prior instance, or the pose configuration of the joint portion triggered and set by the master console prior to that transition.

Specifically, referring to FIG. 10A, when the flexible portion is subjected to the aforementioned switching manner 1), the memory configuration of the joint portion is locally preset as a first memory configuration P2 shown in b of FIG. 10A. When the flexible portion is subjected to the aforementioned switching manner 2), the memory configuration of the joint portion is locally preset as a second memory configuration P3 shown in c of FIG. 10A. When the flexible portion is subjected to the aforementioned switching manner 3), the memory configuration of the joint portion is preset as a third memory configuration P4 shown in d of FIG. 10A.

In another implementation, if the flexible portion is subjected to the aforementioned switching manner 1), and the complete memory configuration of the joint portion is the pose configuration of the joint portion when the flexible portion switches from the second relative positional relationship to the second relationship, or switches to the third relationship, then the memory configuration of the joint portion may also be preset as the second memory configuration or the third memory configuration.

In another implementation, if the flexible portion is subjected to the aforementioned switching manner 2), and the memory configuration of the joint portion is the pose configuration of the joint portion when the flexible portion switches from the second relative positional relationship to the third relationship, then the memory configuration of the joint portion may be preset as the third memory configuration.

Herein, if the previous switching of the flexible portion from the second relative positional relationship to the first relative positional relationship falls into the following situations: 1) the flexible portion switches from the second relative positional relationship to the first relationship (see a in FIG. 10A); and 2) the flexible portion switches from the second relative positional relationship to the second relationship (see a in FIG. 10A). Then, for situation 1), the second memory configuration and the third memory configuration may be set as the straightened configuration when the flexible portion is subjected to the aforementioned switching manner 2) or switching manner 3); and for situation 2), the third memory configuration may be set as the straightened configuration when the flexible portion is subjected to the aforementioned switching manner 3).

Additionally, when the flexible portion is subjected to the aforementioned switching manner 4) to switching manner 6), the operations can be performed in reverse by following the aforementioned configuration setting manner described for switching manner 1) to switching manner 3), which will not be repeated here.

In this embodiment, referring to FIG. 10B, when the flexible portion is subjected to any one of the aforementioned switching manner 1) to switching manner 6), during the gradual switching process of the flexible portion, for instance, when extending a first joint portion 1005 out of the trocar (switching from a in FIG. 10B to b in FIG. 10B), with the gradual extending process, the first joint portion gradually recovers from the straightened configuration P2 to a memory configuration P5. For instance, when extending a second joint portion 1006 out of the trocar (switching from b in FIG. 10B to c in FIG. 10B), with the gradual extending process, the first joint portion gradually recovers from the memory configuration P5 to a memory configuration P6, until it recovers to a final memory configuration P4 (see d in FIG. 10B). When retracting the first joint portion 1005 and the second joint portion 1006 back into the trocar, the reverse operation of extending the first joint portion 1005 and the second joint portion 1006 out of the trocar is performed, transitioning from the memory configuration P6 to the memory configuration P5, until finally transitioning to the straightened configuration P2.

In another implementation, as shown in FIG. 10A, during the gradual switching process of the flexible portion, as the first joint portion and the second joint portion gradually extend out of the trocar, the first joint portion and the second joint portion in the straightened configuration are recovered to the corresponding memory configurations P3 and P4 only after the corresponding first base portion and second base portion completely extend out of the trocar. This enables the transition of the first and second joint portions from the pose configuration P2 to the pose configuration P6.

Exemplarily, when controlling the joint portion to move according to the straightened configuration corresponding to the retraction state, the control information sent in real time includes at least retracting motion information of the surgical tool relative to the central axis. Based on the retracting motion information, a straightening control instruction for adjusting the current configuration of the joint portion to the straightened configuration and a retracting control instruction are configured. The straightening control instruction and the retracting control instruction are sent to the controller, and the controller actuates the joint portion to gradually straighten and retract.

Exemplarily, when controlling the joint portion to move according to the memory configuration corresponding to the restoration state, the control information sent in real time includes at least advancing motion information of the surgical tool relative to the central axis. Based on the advancing motion information, a straightening control instruction for adjusting the current configuration of the joint portion to the memory configuration and a retracting control instruction are configured. The straightening control instruction and the retracting control instruction are sent to the controller, and the controller actuates the joint portion to gradually advance and recover.

In one implementation, when determining the switching situation between the first relative positional relationship and the second relative positional relationship during a motion process of the flexible portion, the switching situation may be determined according to a variation situation of a first axial distance during the motion process of the flexible portion.

In this embodiment, during the motion process of the flexible portion, a magnitude relationship between the first axial distance and the preset distance threshold is determined. If, as the flexible portion moves, the first axial distance changes from being smaller than the preset distance threshold to being greater than the preset distance threshold, it is determined that the flexible portion switches from the second relative positional relationship to the first relative positional relationship. If the first axial distance changes from being greater than the preset distance threshold to being smaller than the preset distance threshold, it is determined that the flexible portion switches from the first relative positional relationship to the second relative positional relationship. By recording each transition in the magnitude relationship between the first axial distance relative to the preset distance threshold, the switching situation can be obtained.

Further, when determining whether the switching situation satisfies the preset condition, it may also be determined by judging whether the variation situation of the first axial distance during the motion process of the flexible portion satisfies the preset condition. If the variation situation of the first axial distance satisfies the preset condition, it is determined that the switching situation of the flexible portion between the first relative positional relationship and the second relative positional relationship satisfies the preset condition.

Herein, the preset condition includes that the current first axial distance is smaller than or equal to the preset distance threshold, and prior to this, there exists the first axial distance being greater than the preset distance threshold. If the variation situation of the first axial distance satisfies the preset condition, it is determined that the current motion state of the flexible portion is the retraction state or the restoration state relative to the trocar.

In one implementation, when the operation state has just entered the retraction state or the restoration state, the control information may be an instantaneous control instruction to control a distal portion to automatically move to a preset pose configuration. For example, a one-touch retract or one-touch restore button is configured on the master console, and the doctor touches the button to realize the one-touch retraction of the flexible portion into the trocar, or the one-touch extension out of the trocar.

Herein, each instantaneous control instruction may only set the pose configuration for a single joint mechanism. That is, each time the button is touched, one instantaneous control instruction is output, and each time, one joint mechanism is controlled to automatically move to the preset pose configuration. When the button is touched again, the next joint mechanism is controlled to automatically move to the preset pose configuration.

In another implementation, the operation state is a continuous retraction/restoration state, the control information is a continuous control instruction, and the distal portion is controlled to continuously move according to the preset pose configuration during a continuous sending of the continuous control instruction. For example, if the pose configuration is the straightened configuration during the retraction state, then with the continuous control instruction, the flexible portion/joint mechanism straightens at t1-t2, and retracts at t2-t3. If the pose configuration is the memory configuration during the restoration state, then the flexible portion/joint mechanism advances at t1-t2, and restores at t2-t3. Or, a portion of the flexible portion exiting the trocar is determined based on the exiting amount, and this portion is straightened in real time according to the straightened configuration; and a portion of the distal portion extending out of the trocar is determined based on the advancing amount, and this portion is restored in real time according to the memory configuration.

### V. Sleeve-Hiding Function - Cooperative Motion

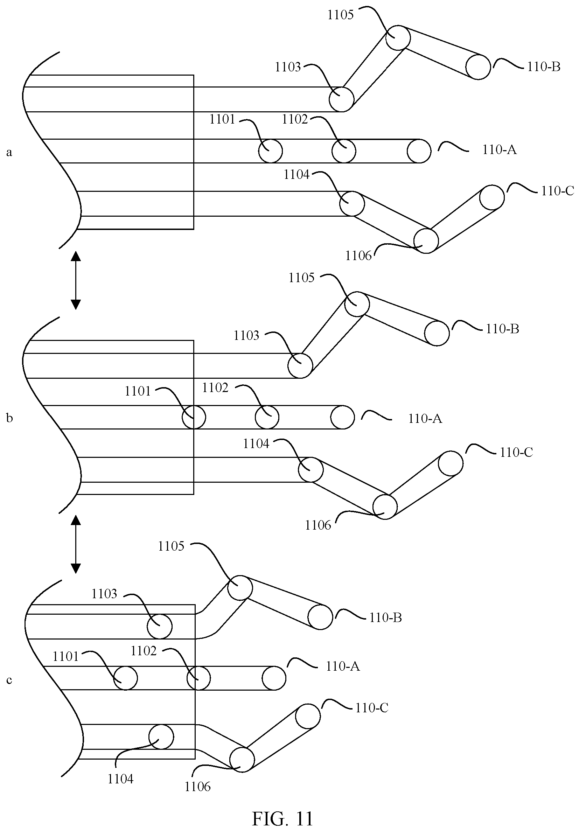

Referring to FIG. 11, a fourth embodiment of a control method for a surgical robot is provided in the present application, which is specifically illustrated as follows:
The surgical tool includes an endoscope and at least one surgical instrument, a corresponding relationship is established between a joint mechanism of the endoscope and a joint mechanism of the at least one surgical instrument, and when determining a target joint mechanism in a retraction state or a restoration state among the endoscope or the at least one surgical instrument, another joint mechanism having the corresponding relationship with the target joint mechanism is controlled to perform a cooperative motion.

In this embodiment, when the flexible portion of one of the endoscope and the surgical instrument switches between the first relative positional relationship and the second relative positional relationship with respect to the trocar, and prior to this, there exists a switch from the second relative positional relationship to the first relative positional relationship, it is determined that this surgical tool is in the retraction state or the restoration state.

In the retraction state or the restoration state, as the flexible portion of the surgical instrument or the endoscope advances or retracts, a significant change in the pose configuration will occur, resulting in substantial alterations in the position of the surgical instrument relative to the field of view of the endoscope. Under such substantial change, the surgical instrument is prone to exiting the field of view of the endoscope, thereby losing the visual position of the surgical instrument. Therefore, in this case, the other surgical instrument or the endoscope that is not in the retraction state or the restoration state is controlled to perform cooperative motion accordingly, so that the difference in the variation amplitude of the pose configuration between the two is small, reducing the probability of the surgical instrument exiting the field of view of the endoscope.

Specifically, when establishing the corresponding relationship between the joint mechanism of the surgical instrument and the joint mechanism of the endoscope, since the surgical instrument is typically positioned more distally relative to the endoscope, the camera at the distal end of the endoscope can capture the end effector of the more distal surgical instrument. Based on this, it can be determined that the first joint mechanism and the second joint mechanism of the surgical instrument are located at more distal positions than the first joint mechanism and the second joint mechanism of the endoscope. Moreover, a first corresponding relationship between the first joint mechanism of the endoscope and the second joint mechanism of the surgical instrument, and a second corresponding relationship between the second joint mechanism of the endoscope and the second joint mechanism of the surgical instrument are predefined. Based on the first corresponding relationship and the second corresponding relationship, the second joint mechanism of the surgical instrument is controlled to move along with the first joint mechanism and the second joint mechanism of the endoscope.

Herein, the switching of the flexible portion of the endoscope between the first relative positional relationship and the second relative positional relationship includes a sequential switching from the second relative positional relationship to the first relationship, the second relationship, and the third relationship respectively, specifically including a forward or reverse sequential switching. The switching from the second relative positional relationship to the second relationship may be divided into two switching stages, namely, from the second relative positional relationship to the first relationship, and from the first relationship to the second relationship. The switching from the second relative positional relationship to the third relationship may be divided into three switching stages, namely, from the second relative positional relationship to the first relationship, from the first relationship to the second relationship, and from the second relationship to the third relationship.

Exemplarily, as shown in FIG. 11, an endoscope 110-A, a surgical instrument 110-B, and a surgical instrument 110-C are included. Corresponding relationships of the second joint mechanism 1101 and the first joint mechanism 1102 of the endoscope 110-A associated with the second joint mechanism 1103 of the surgical instrument 110-B and the second joint mechanism 1104 of the surgical instrument 110-C, are respectively established. A first corresponding relationship is that, as the second joint mechanism 1101 retracts into the trocar, the second joint mechanism 1103 and the second joint mechanism 1104 retract. A second corresponding relationship is that, as the first joint mechanism 1102 retracts into the trocar, the second joint mechanism 1103 and the second joint mechanism 1104 retract into the trocar.

Specifically, continuing to refer to FIG. 11, an initial relative positional relationship among the respective joint mechanisms among the endoscope 110-A, the surgical instrument 110-B, and the surgical instrument 110-C are as shown in a of FIG. 11. When the second joint mechanism 1101 moves from the position shown in a of FIG. 11 to a position where the second joint mechanism 1101 begins to enter the trocar (as shown in b of FIG. 11), then according to the first corresponding relationship, the second joint mechanism 1103 and the second joint mechanism 1104 may also begin to retract. When the first joint mechanism 1002 moves from the position shown in b of FIG. 11 to a position where the first joint mechanism 1002 begins to enter the trocar (as shown in c of FIG. 11), then according to the second corresponding relationship, the second joint mechanism 1103 and the second joint mechanism 1104 begin to retract into the trocar.

In one implementation, when controlling the second joint mechanism 1103 and the second joint mechanism 1104 to retract according to the first corresponding relationship, on the one hand, the second joint mechanism 1103 and the second joint mechanism 1104 are constrained from retracting into the trocar; on the other hand, the second joint mechanism 1103 and the second joint mechanism 1104 may be constrained to retract according to a moving distance of the second joint mechanism 1101, or the second joint mechanism 1103 and the second joint mechanism 1104 are constrained to retract to a position just before retracting into the trocar.

In one implementation, when controlling the second joint mechanism 1103 and the second joint mechanism 1104 to gradually retract according to the first corresponding relationship, the second joint mechanism 1101 stays at the position where second joint mechanism 1101 begins to enter the trocar, until the second joint mechanism 1103 and the second joint mechanism 1104 retract by a preset distance, or the distance to the trocar port is within a preset distance threshold.

In one implementation, when controlling the second joint mechanism 1103 and the second joint mechanism 1104 to gradually retract according to the first corresponding relationship, the second joint mechanism 1101 is controlled, according to the input control information, to gradually retract into the trocar at a retraction rate of a preset weight, enabling the first joint mechanism 1102, the second joint mechanism 1103, and the second joint mechanism 1104 to simultaneously reach the position where they begin to enter the trocar.

In one implementation, when controlling the second joint mechanism 1103 and the second joint mechanism 1104 to retract into the trocar according to the second corresponding relationship, the first joint mechanism 1102 is controlled, according to the input control information, to gradually retract into the trocar at a retraction rate of a preset weight, enabling the first joint mechanism 1102, the second joint mechanism 1103, and the second joint mechanism 1104 to simultaneously and completely retract into the trocar.

Additionally, after the first joint mechanism 1102, the second joint mechanism 1103, and the second joint mechanism 1104 completely retract into the trocar, as the endoscope 110-A continues to retract, the first joint mechanism 1105 of the surgical instrument 110-B and the first joint mechanism 1106 of the surgical instrument 110-C also gradually retract into the trocar.

In another implementation, the first joint mechanism 1105 and the first joint mechanism 1106 may remain outside the trocar to continue performing surgical operations, which are applicable to operations in a narrow surgical space or the treatment of a lesion relatively close to the body surface.

### VI. Sleeve-Hiding Function - View Switching

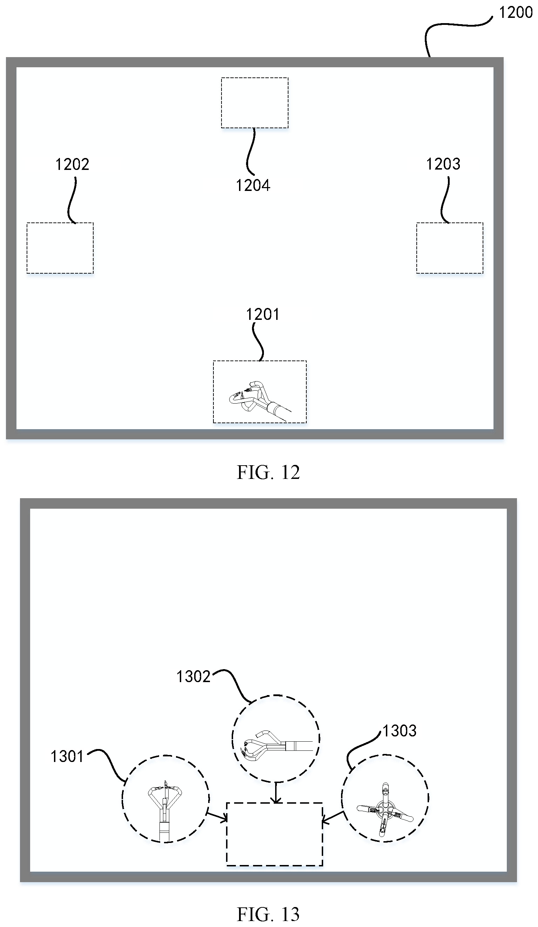

Referring to FIGS. 12 to 13, a fifth embodiment of a control method for a surgical robot is provided in the present application, which is specifically illustrated as follows:
The remote center of motion is provided with a third coordinate system. The number of surgical tools is multiple. The surgical robot also includes a display device. Based on the third coordinate system, a kinematic model is established for each surgical tool. Based on the kinematic model, during the motion process of the flexible portion of each surgical tool, a third relative positional relationship of the joint base and the end effector among the respective surgical tools is determined. Based on the third relative positional relationship, a three-dimensional model diagram of the flexible portion corresponding to each surgical tool is generated. The three-dimensional model diagram is displayed on the display device. Or, according to a switching situation of the flexible portion between the first relative positional relationship and the second relative positional relationship, a two-dimensional view from a corresponding viewing angle is captured from the three-dimensional model diagram, and the two-dimensional view is displayed on the display device.

In this embodiment, referring to FIG. 5B, for multiple surgical tools, for example, in addition to the first base portions of the endoscope 11-A and the surgical instrument 11-B being respectively provided with a first coordinate system XewYewZew and XiwYiwZiw, and the second base portions of the endoscope 11-A and the surgical instrument 11-B being respectively provided with a second coordinate system XebYebZeb and XibYibZib, a third coordinate system xmymzm is also provided at the remote center of motion, and the third coordinate system xmymzm serves as a position reference datum for estimating the relative positions among the first coordinate systems XewYewZew, XiwYiwZiw and the second coordinate systems XebYebZeb, XibYibZib during the motion process of the flexible portions.

Specifically, taking the third coordinate system xmymzm as the position reference datum, by establishing kinematic models for the surgical instruments and the endoscope, the third relative positional relationships among the first base portions, the second base portions, and the end effectors between the surgical instrument and the surgical instrument, and between the surgical instrument and the endoscope are obtained, so that the motion process of each surgical tool can be displayed dynamically in real time through a UI view. Herein, the UI view includes a three-dimensional model diagram and a two-dimensional view, which are usually presented on the screen of the master console, and may also be presented on the surgical robot and/or the imaging cart.

As shown in FIG. 12, the display area of the UI view may be a display area 1201 at the bottom of the screen of the display interface 1200, a display area 1202 on the left side of the screen, a display area 1203 on the right side of the screen, or a display area 1204 at the top of the screen, or any other position in the display interface, so as to facilitate the doctor to observe the relative motion positions among the respective surgical tools in the display interface. Here, the display area of the UI view is located at the position of the bottom of the screen 1201 by default.

Further, based on the switching situation of the flexible portion between the first relative positional relationship and the second relative positional relationship, the respective execution processes of the flexible portion may be determined. Then, according to the different execution processes of the flexible portion, the two-dimensional view from the associated viewing angle is captured from the three-dimensional model diagram, so as to more clearly and plainly display the relative motion positions of the respective surgical tools in the different execution processes of the flexible portion. Herein, the two-dimensional views of different viewing angles include at least a top view, a side view, and a front view.

In one implementation, when determining the respective execution processes of the flexible portion based on the switching situation of the flexible portion between the first relative positional relationship and the second relative positional relationship, if the flexible portion gradually switches from the second relative positional relationship to the first relative positional relationship and then gradually switches from the first relative positional relationship to the second relative positional relationship, then it is determined that the flexible portion is in a first execution process. If the flexible portion, after switching from the second relative positional relationship to the first relative positional relationship, continuously stays in the first relative positional relationship, then it is determined that the flexible portion is in a second execution process. Before the master-slave control is activated (when the flexible portion is in the second relative positional relationship for the first time), it is determined that the flexible portion is in a third execution process.

Further, referring to FIG. 13, when the flexible portion is in the first execution process, a top view 1301 of the three-dimensional model diagram is captured. When the flexible portion is in the second execution process, a side view 1302 of the three-dimensional model diagram is captured. When the flexible portion is in the third execution process, a front view 1303 of the three-dimensional model diagram is captured. In order to prevent the two-dimensional view from obscuring the surgical field of view in the display interface as much as possible, usually one two-dimensional view is displayed in the display area, and when the flexible portion switches among the first, second, and third execution processes, the captured top view 1301, side view 1302, and front view 1303 are automatically switched and displayed. The three two-dimensional views also allow manual selection and switching through touch screen buttons on the master console, the surgical robot, and/or the imaging cart.

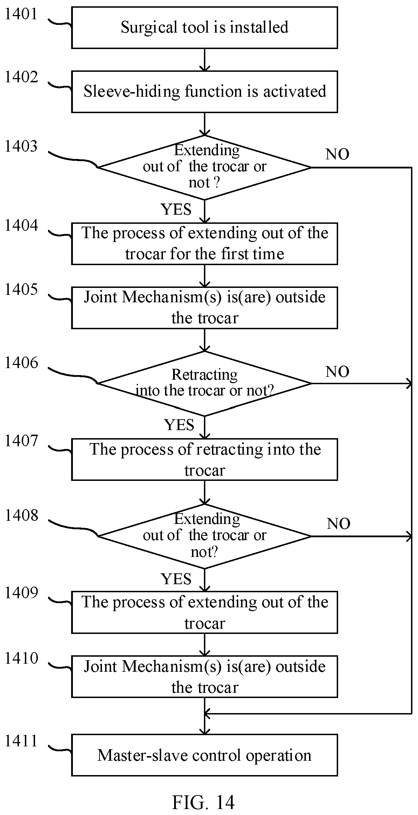

The above has described in detail the sleeve-hiding function of the master-slave operation, and the corresponding resetting of the remote center of motion under the sleeve-hiding function, the retraction and restoration of the surgical tool relative to the trocar, the cooperative motion of multiple surgical tools, and the view switching under different execution processes of the surgical tool in the control method for the surgical robot. Next, combining the aforementioned several embodiments regarding the control method for the surgical robot, a preferred embodiment of a complete process is provided, referring to FIG. 14, which is specifically shown as follows:
For steps 1401 to 1402, the surgical tool is installed on the controller and inserted into the patient's body through the trocar arranged at the human body incision. Meanwhile, to ensure the safety of the surgical tool operation, the sleeve-hiding function needs to be activated before the master-slave operation, so as to allow the use of relevant algorithms to execute the control method for the surgical robot, and if the activation is unsuccessful, the control signal for the master-slave operation is in a disconnected state, rendering the surgical tool uncontrollable, that is, the surgical robot does not respond to the control information input from the master console to execute the surgical action. After the sleeve-hiding function is successfully activated, the master console can control the surgical tool to move.
For step 1403, if the action of extending out of the trocar is not performed on the surgical tool, then when performing the master-slave control operation in step 1411 on the surgical tool, in response to the control information output from the master console, it is determined that the surgical tool is in the second relative positional relationship with respect to the trocar. At this point, motion control is performed on the surgical tool based on the first constraint. That is, the surgical tool is only permitted to perform a rotational motion around its own central axis (or further perform the clamping opening and closing motion of the end effector), while the wrist joint (the first joint portion) and the shoulder-elbow joint (the second joint portion) do not perform the bending action and still remain in a straightened state.
For steps 1404 to 1405, if the action of extending out of the trocar is performed on the surgical tool, then the relative positional relationship between the flexible portion and the trocar is further detected during the process of the surgical tool extending out of the trocar, so as to determine the motion constraint of the flexible portion, specifically including the following situations:
   A. During the translation motion of the surgical tool along the central axis of the trocar towards the outside of the trocar, if the first axial distance between the first coordinate system XwYwZw of the wrist base (the first base portion) and the remote center of motion in the direction of the central axis of the trocar is smaller than the preset distance threshold f, then the wrist joint (the first joint portion) and the shoulder-elbow joint (the second joint portion) still maintain the straightened state and do not perform the bending motion, effectively avoiding the collision between the wrist joint and the port position of the trocar. That is, the relative positional relationship is the second relative positional relationship, and the motion constraint is the second constraint.
   B. If the first axial distance corresponding to the wrist base is greater than or equal to f, and simultaneously the first axial distance between the second coordinate system XbYbZb of the shoulder-elbow base (the second base portion) and the remote center of motion in the direction of the central axis of the trocar is smaller than f, then the master-slave control operation can realize the rotation motion of the wrist joint in two degrees of freedom, thereby adjusting the pose of the end effector. Meanwhile, the shoulder-elbow joint still remains in the straightened state and does not perform the bending motion, thus effectively avoiding the collision between the shoulder-elbow joint and the port position of the trocar. That is, the relative positional relationship is the second relationship, and the motion constraint is the second degree-of-freedom constraint.
   C. If the first axial distance corresponding to the shoulder-elbow base is greater than or equal to f, both the shoulder-elbow joint and the wrist joint are entirely located outside the trocar, enabling rotational motion in two degrees of freedom respectively. Under master-slave control operation, the motion in six degrees of freedom of the surgical tool can be achieved. That is, the relative positional relationship is the third relationship, and the motion constraint is the third degree-of-freedom constraint.
For steps 1406 to 1407, if the first axial distance corresponding to the shoulder-elbow base in step 1406 is equal to f, then the surgical tool in step 1407 is executed to begin retracting into the trocar, if the first axial distance corresponding to the shoulder-elbow base is smaller than f, the shoulder-elbow joint performs a straightening action gradually parallel to the central axis of the trocar, and when the first axial distance corresponding to the wrist base is equal to f, the shoulder-elbow joint is completely straightened, while the wrist joint can still perform the bending motion according to the master-slave control operation.

As the first axial distance corresponding to the wrist base is gradually smaller than f, the wrist joint performs the straightening action gradually parallel to the central axis of the trocar, until the wrist joint is completely straightened. When the wrist joint performs the straightening action, the shoulder-elbow joint still maintains the straightened state. At this time, the linkage in the long-axis schematic diagram is allowed to perform the rotation motion around its own axis.

For steps 1408 to 1410, the corresponding motions are executed according to the operable sequence of the wrist joint and the shoulder-elbow joint in step 1404, during the process of the surgical tool extending out of the trocar, the wrist joint and the shoulder-elbow joint automatically execute the restoration to the pose configuration before retracting into the trocar in step 1407, until the shoulder-elbow joint is completely outside the trocar, and then a six-degree-of-freedom master-slave control operation is executed. The execution of step 1410 is as described in step 1405, which will not be repeated here.

In one implementation, step 1407 and step 1409 may execute force feedback prompts at the master manipulator of the master console by establishing a mechanical model, and/or on an observation window of the display interface of the master console, by adding a stroke progress bar of the motion of the surgical tool along the central axis of the trocar, and/or by other means, to prompt the doctor that the surgical tool is in the process of retracting into the trocar (step 1407) or extending out of the trocar for the second time (step 1409).

Exemplarily, the force feedback prompt may be a vibration prompt at the master manipulator, or may be a spring force prompt at the master manipulator. The spring force prompt refers to being realized by performing inverse kinematics calculation according to the force feedback at the master manipulator.

Furthermore, when presenting the two-dimensional views of multiple surgical tools in the display interface, it is preset to show the top view when the surgical robot is controlled to perform steps 1401-1402, show the side view when performing steps 1404, 1407, and 1409, and show the front view when performing steps 1405, 1410, and 1411.

It should be noted that other sequencing schemes that may be easily conceived by persons skilled in the art within the technical scope disclosed in the present application should also fall within the protection scope of the present application, which will not be elaborated one by one here.

Persons skilled in the art can clearly understand that, for the convenience and conciseness of description, only the division of the above functional modules is used as an example for illustration. In practical applications, the above functions may be allocated to different functional units and modules for completion as needed. That is, the internal structure of the mobile terminal is divided into different functional units or modules to complete all or part of the functions described above. The respective functional modules in the embodiments may be integrated into one processing unit, or each unit may exist physically alone, or two or more units may be integrated into one unit. The above integrated units may be implemented in the form of hardware, or in the form of software functional units. In addition, the specific names of the respective functional modules are only for the convenience of distinguishing each other, and are not used to limit the protection scope of the present application. For the specific working process of the modules in the above mobile terminal, reference may be made to the corresponding process in the foregoing method embodiments, which will not be repeated here.

An embodiment of the present application also provides a computer-readable storage medium. The computer-readable storage medium stores a computer program, and the computer program, when executed by a processor, causes the steps in the above respective method embodiments to be implemented.

An embodiment of the present application provides a computer program product. When the computer program product runs on a mobile terminal, the mobile terminal is caused to implement the steps in the above respective method embodiments upon executing the computer program product.

It should be understood that, when used in the specification and the appended claims of the present application, the term "comprising/including" indicates the presence of the described features, integers, steps, operations, elements, and/or components, but does not exclude the presence or addition of one or more other features, integers, steps, operations, elements, components, and/or collections thereof.

It should also be understood that the term "and/or" used in the specification and the appended claims of the present application refers to any combination and all possible combinations of one or more of the associated listed items, and includes these combinations.

As used in the specification and the appended claims of the present application, the term "if" may be interpreted as "when" or "upon" or "in response to determining" or "in response to detecting" depending on the context. Similarly, the phrase "if determined" or "if the described condition or event is detected" may be interpreted as meaning "upon determining" or "in response to determining" or "upon detecting the described condition or event" or "in response to detecting the described condition or event" depending on the context.

In addition, in the description of the specification and the appended claims of the present application, the terms "first", "second", "third", etc. are only used for distinguishing description, and cannot be understood as indicating or implying relative importance.

In the description of the specification of the present application, reference to the terms "one embodiment" or "some embodiments," etc., means that a particular feature, structure, or characteristic described in connection with the embodiment is included in one or more embodiments of the present application. Thus, the appearances of the phrases "in an embodiment", "in some embodiments", "in some other embodiments", "in still other embodiments", etc. in various places throughout this specification are not necessarily all referring to the same embodiment, but mean "one or more but not all embodiments", unless otherwise specifically emphasized. The terms "comprising", "including", "having" and their variations all mean "including but not limited to", unless otherwise specifically emphasized.

Persons skilled in the art can clearly understand that, for the convenience and conciseness of description, only the division of the above functional units and modules is used as an example for illustration. In practical applications, the above functions may be allocated to different functional units and modules for completion as needed. That is, the internal structure of the device is divided into different functional units or modules to complete all or part of the functions described above. The respective functional units and modules in the embodiments may be integrated into one processing unit, or each unit may exist physically alone, or two or more units may be integrated into one unit. The above integrated units may be implemented in the form of hardware, or in the form of software functional units. In addition, the specific names of the respective functional units and modules are only for the convenience of distinguishing each other, and are not used to limit the protection scope of the present application. For the specific working process of the units and modules in the above system, reference may be made to the corresponding process in the foregoing method embodiments, which will not be repeated here.

In the above embodiments, the description of each embodiment has its own emphasis. For a part that is not described in detail or recorded in a certain embodiment, reference may be made to related descriptions in other embodiments.

A person of ordinary skill in the art can realize that the units and algorithm steps of the respective examples described in connection with the embodiments disclosed herein can be implemented by electronic hardware, or a combination of computer software and electronic hardware. Whether these functions are executed by hardware or software depends on the specific application and design constraint conditions of the technical solution. Professional technicians may use different methods to implement the described functions for each specific application, but such implementation should not be considered to go beyond the scope of the present application.

In addition, the respective functional units in the respective embodiments of the present application may be integrated into one processing unit, or each unit may exist physically alone, or two or more units may be integrated into one unit. The above integrated units may be implemented in the form of hardware, or in the form of software functional units.

The integrated module/unit, if implemented in the form of a software functional unit and sold or used as an independent product, may be stored in a computer-readable storage medium. Based on such understanding, the implementation of all or part of the processes in the methods of the above embodiments in the present application may also be completed by instructing related hardware through a computer program. The computer program may be stored in a computer-readable storage medium. The computer program, when executed by a processor, enables the steps of the above respective method embodiments to be implemented. Herein, the computer program includes computer program codes, and the computer program codes may be in the form of source code, object code, executable file, or some intermediate forms, etc. The computer-readable medium may include: any entity or device capable of carrying the computer program code, a recording medium, U disk, a mobile hard disk, a magnetic disk, an optical disk, a computer memory, a read-only memory (ROM, Read-Only Memory), a random-access memory (RAM, Random Access Memory), an electrical carrier signal, a telecommunication signal, and a software distribution medium, etc. It should be noted that the content contained in the computer-readable medium may be appropriately increased or decreased according to the requirements of legislation and patent practice in the jurisdiction. For example, in some jurisdictions, according to legislation and patent practice, the computer-readable medium does not include electrical carrier signals and telecommunication signals.

The above embodiments are only used to illustrate the technical solutions of the present application, and are not intended to limit the present application. Although the present application has been described in detail with reference to the foregoing embodiments, persons of ordinary skill in the art should understand that: they can still modify the technical solutions recorded in the foregoing respective embodiments, or make equivalent replacements to some of the technical features therein. These modifications or replacements do not make the essence of the corresponding technical solutions depart from the spirit and scope of the technical solutions of the respective embodiments of the present application, and thus should all be included within the protection scope of the present application.

## Claims

1. A surgical robot system, wherein the surgical robot system comprises a master console and a surgical robot, wherein the surgical robot comprises a controller, a surgical tool and a trocar, and the master console is communicatively connected to the surgical robot;
the surgical tool is extendably accommodated in the trocar, and comprises a flexible portion at a distal end, the flexible portion comprises at least one joint mechanism, and the joint mechanism comprises a base portion and a joint portion;
the controller is configured to execute motion control of the surgical tool, comprising:
determining, in response to control information for the surgical tool sent from the master console, whether the flexible portion and the trocar are in a first relative positional relationship or a second relative positional relationship;
performing motion control on the flexible portion according to the control information and in accordance with a preset first constraint associated with the first relative positional relationship; or,
performing motion control on the flexible portion according to the control information and in accordance with a preset second constraint associated with the second relative positional relationship; wherein a length of the flexible portion accommodated within the trocar is shorter in the first relative positional relationship than in the second relative positional relationship, and when the length of the flexible portion accommodated in the trocar is shorter, a number of joint mechanisms constrained for the flexible portion is greater, and a constraint on the joint mechanism comprises a constraint on a motion degree of freedom of the joint portion.

2. The surgical robot system according to claim 1, wherein the surgical tool further comprises an end effector, the joint mechanism comprises a first joint mechanism and a second joint mechanism, the first joint mechanism comprises a first base portion and a first joint portion, the second joint mechanism comprises a second base portion and a second joint portion, the second joint portion is coupled with the first base portion, and the first joint portion is coupled with the end effector;
the determining whether the flexible portion and the trocar are in the first relative positional relationship or the second relative positional relationship comprises:
determining whether at least one of the end effector, the first base portion and the second base portion satisfies a preset positional relationship relative to a port position of the trocar;
determining that the flexible portion and the trocar are in the first relative positional relationship if at least one of the end effector, the first base portion and the second base portion satisfies a preset positional relationship; and
determining that the flexible portion and the trocar are in the second relative positional relationship if none of the end effector, the first base portion and the second base portion satisfies the preset positional relationship.

3. The surgical robot system according to claim 2, wherein the first constraint comprises a first degree-of-freedom constraint, a second degree-of-freedom constraint and a third degree-of-freedom constraint;
the performing motion control on the flexible portion according to the control information and in accordance with the preset first constraint associated with the first relative positional relationship comprises:
performing motion control on the flexible portion according to the control information and in accordance with the first degree-of-freedom constraint if the end effector satisfies the preset positional relationship, and the first base portion does not satisfy the preset positional relationship;
performing motion control on the flexible portion according to the control information and in accordance with the second degree-of-freedom constraint if the first base portion satisfies the preset positional relationship, and the second base portion does not satisfy the preset positional relationship;
performing motion control on the flexible portion according to the control information and in accordance with the third degree-of-freedom constraint if the second base portion satisfies the preset positional relationship.

4. The surgical robot system according to claim 3, wherein the first degree-of-freedom constraint comprises: a motion of the end effector to execute a functional operation, and a rotation motion of the first joint portion and the second joint portion around their own central axes, and a translation motion a rotation motion along a central axis of the trocar;
the second degree-of-freedom constraint comprises: the first degree-of-freedom constraint, and a bending motion of the first joint portion along the first base portion; and
the third degree-of-freedom constraint comprises: at least one of the first degree-of-freedom constraint and the second degree-of-freedom constraint, and a bending motion of the second joint portion along the second base portion.

5. The surgical robot system according to claim 2, wherein the second constraint comprises: a rotation motion of the end effector, the first joint portion and the second joint portion around their own central axes, and a rotation motion and a translation motion along a central axis of the trocar.

6. The surgical robot system according to claim 2, wherein the first base portion is provided with a first coordinate system, the second base portion is provided with a second coordinate system, a remote center of motion is disposed on a central axis of the trocar, and a preset distance threshold is set for the remote center of motion relative to the port position;
the determining whether at least one of the end effector, the first base portion and the second base portion satisfies the preset positional relationship relative to the port position of the trocar comprises:
determining first axial distances in a distal direction of the central axis of the trocar between the first coordinate system and the remote center of motion, and between the second coordinate system and the remote center of motion, respectively;
comparing a magnitude between each of the first axial distances and the preset distance threshold, and based on a comparison result, respectively determining whether the end effector, the first base portion and the second base portion satisfy the preset positional relationship relative to the port position of the trocar.

7. The surgical robot system according to claim 6, wherein the control method further comprises:
obtaining size information of the flexible portion, and determining a second axial distance between the remote center of motion and a target lesion region;
controlling, based on each of the size information and the second axial distance, the trocar to perform a translation motion relative to the central axis; and
resetting, in response to a setting instruction for the remote center of motion, the remote center of motion according to a termination position of the trocar when performing the translation motion.

8. The surgical robot system according to claim 7, wherein the controlling, based on each of the size information and the second axial distance, the trocar to perform the translation motion relative to the central axis comprises:
determining, according to the size information, respective segment lengths of the end effector, the first base portion and the second base portion;
setting, according to each of the segment lengths and the second axial distance, a translation range of the trocar relative to the central axis of the trocar, and controlling the trocar to move within the translation range; or,
setting, according to each of the segment lengths and the second axial distance, respective translation nodes of the trocar relative to the central axis of the trocar, and controlling the trocar to move at each of the translation nodes, such that the termination position of the trocar is located at one of the translation nodes.

9. The surgical robot system according to claim 7, wherein the resetting, in response to the setting instruction for the remote center of motion, the remote center of motion according to the termination position of the trocar when performing the translation motion comprises:
determining, in response to the setting instruction for the remote center of motion, a translation distance of the trocar according to the termination position of the trocar when performing the translation motion;
controlling the remote center of motion to move according to the translation distance, to obtain a reset remote center of motion, wherein the remote center of motion is located within a position range where the trocar is located.

10. The surgical robot system according to claim 1, wherein the controller is further configured to perform operations of:
determining a switching situation of the flexible portion between the first relative positional relationship and the second relative positional relationship during a motion process; and
determining, according to the switching situation and the control information, a current motion state of the flexible portion, and controlling the joint portion to move according to a joint pose configuration corresponding to the motion state.

11. The surgical robot system according to claim 10, wherein the determining, according to the switching situation and the control information, the current motion state of the flexible portion comprises:
determining whether the switching situation satisfies a preset condition, wherein the preset condition comprises that the flexible portion currently performs a gradual switching between the first relative positional relationship and the second relative positional relationship, and prior to this, there exists a switching of the flexible portion from the second relative positional relationship to the first relative positional relationship; and
determining, if the switching situation satisfies the preset condition, the current motion state of the flexible portion according to the control information.

12. The surgical robot system according to claim 11, wherein the controlling the joint portion to move according to the joint pose configuration corresponding to the motion state comprises:
setting, when the control information is an advancing control, the joint pose configuration corresponding to the motion state as a memory configuration of the joint portion when the flexible portion is about to switch from the first relative positional relationship to the second relative positional relationship;
setting, when the control information is a retracting control, the joint pose configuration corresponding to the motion state as a straightened configuration of the joint portion; and
controlling the joint portion to move according to the memory configuration or the straightened configuration.

13. The surgical robot system according to claim 1, wherein the surgical tool comprises an endoscope and at least one surgical instrument, a corresponding relationship is established between a joint mechanism of the endoscope and a joint mechanism of the at least one surgical instrument, and the controller is further configured to:
control, when determining a target joint mechanism in a retraction state or a restoration state among the endoscope or the at least one surgical instrument, another joint mechanism having the corresponding relationship with the target joint mechanism to perform a cooperative motion.

14. The surgical robot system according to claim 1, wherein a remote center of motion is disposed on a central axis of the trocar, and the remote center of motion is provided with a third coordinate system; a number of surgical tools is multiple; the surgical robot further comprises a display device; and the controller is further configured to:
establish a kinematic model for each of the surgical tools based on the third coordinate system;
determine, during a motion process of the flexible portion for each of the surgical tools, a third relative positional relationship of a joint base and an end effector among the surgical tools based on the kinematic model;
generate a three-dimensional model diagram of the flexible portion corresponding to each of the surgical tools based on the third relative positional relationship;
display the three-dimensional model diagram on the display device; or,
capture a two-dimensional view from a corresponding viewing angle in the three-dimensional model diagram according to a switching situation of the flexible portion between the first relative positional relationship and the second relative positional relationship, and display the two-dimensional view on the display device.
